# EUROPÄISCHE PATENTANMELDUNG

(11) **EP 2 110 443 A1**
(43) Veröffentlichungstag der Anmeldung: **21.10.2009**
(21) Anmeldenummer: 08154487.6
(22) Anmeldetag: 14.04.2008
(51) Int. Cl.: C12Q 1/68

(54) **FNTB Promotorpolymorphismen**

(71) Anmelder: Universität Duisburg-Essen, 47048 Duisburg (DE)
(72) Erfinder: Bachmann, Hagen-Sjard, Dr., 45478 Mülheim/Ruhr (DE); Siffert, Winfried, Prof. Dr., 45884 Gelsenkirchen (DE)
(74) Vertreter: Helbing, Jörg

(57) **Zusammenfassung**

Die Erfindung betrifft ein Verfahren zur Vorhersage des Verlaufs von Erkrankungen wie z. B. Krebserkrankungen, Herz-Kreislauf-Erkrankungen, parasitärer Erkrankungen und Progerie-Syndrome, zur Vorhersage des Ansprechens auf pharmakologische oder nicht-pharmakologische Therapiemaßnahmen, zur Behandlung dieser Erkrankungen sowie zur Vorhersage unerwünschter Arzneimittelwirkungen umfassend den Nachweis von Genveränderungen im humanen Gen *FNTB,* das für die β-Untereinheit des Proteins Farnesyltransferase kodiert. Die Genveränderungen bestehen vorzugsweise darin, dass in der Genpromotorregion von *FNTB* an den Position -609, -179 und -173 Polymorphismen vorliegen. Bei Position -609 wird ein Guanin durch ein Cytosin und bei Position -179 ein Thymin durch ein Adenin substituiert. An Position - 173 kommt es zur Deletion eines Guanin. Mit Hilfe der genannten Genveränderungen können weitere für die oben genannten Zwecke verwendbare Genveränderungen detektiert und validiert werden. Die Erfindung betrifft weiterhin Kits, die für diese Verfahren geeignet sind.

## Beschreibung

Die Erfindung betrifft ein Verfahren zur Vorhersage des Verlaufs von Erkrankungen wie z. B. Krebserkrankungen, Herz-Kreislauf-Erkrankungen, parasitärer Erkrankungen und Progerie-Syndrome, zur Vorhersage des Ansprechens auf pharmakologische oder nicht-pharmakologische Therapiemaßnahmen, zur Behandlung dieser Erkrankungen sowie zur Vorhersage unerwünschter Arzneimittelwirkungen umfassend den Nachweis von Genveränderungen im humanen Gen *FNTB,* das für die β-Untereinheit des Proteins Farnesyltransferase kodiert. Die Genveränderungen bestehen vorzugsweise darin, dass in der Genpromotorregion von *FNTB* an den Position -609, -179 und -173 Polymorphismen vorliegen. Bei Position -609 wird ein Guanin durch ein Cytosin und bei Position -179 ein Thymin durch ein Adenin substituiert. An Position - 173 kommt es zur Deletion eines Guanin. Mit Hilfe der genannten Genveränderungen können weitere für die oben genannten Zwecke verwendbare Genveränderungen detektiert und validiert werden. Die Erfindung betrifft weiterhin Kits, die für diese Verfahren geeignet sind.

### Hintergrund der Erfindung

Ein wesentlicher Aspekt bei der Behandlung von Erkrankungen ist zunächst das Erstellen einer genauen Diagnose. Dies führt zu Kenntnissen darüber, in welchem Stadium sich die Erkrankung befindet, was auch über die nachfolgenden Therapiemodalitäten entscheidet, z. B. chirurgische Intervention, Strahlentherapie oder pharmakologische Therapie, eventuell auch unter Einsatz physikalischer Verfahren oder Umstellung der Ernährung im Sinne einer spezifischen Ernährung, z. B. für Herz-Kreislauf-Patienten, Krebspatienten oder Patienten mit Stoffwechselstörungen.

Bei Krebspatienten zum Beispiel beinhaltet das klassische Verfahren zur Diagnosestellung das sogenannte Staging" und Grading". Das Staging beschreibt die Tumorgröße und die Invasivität und untersucht, ob Lymphknoten befallen sind und ob Fernmetastasen vorhanden sind (z. B. TNM-System). Beim Grading werden die Tumorzellen histologisch untersucht, wobei hoch differenzierten Tumorzellen ein niedrigeres Malignitätspotential zugeordnet wird als schwach oder entdifferenzierten Zellen. Obwohl dieses System sich in der Praxis bewährt hat, ist die individuelle Vorhersagekraft für den einzelnen Patienten limitiert, und es entspricht der klinischen Erfahrung, dass Patienten mit gleichem Tumorstadium deutlich unterschiedliche Krankheitsverläufe zeigen und unterschiedlich auf eine Therapie ansprechen, was sich letztendlich in drastisch unterschiedlichen Überlebenszeiten zeigt. Aus diesem Grunde ist es für die klinische Praxis erforderlich, zusätzliche und präzisere Marker bereitzustellen, die eine verbesserte Individualprognose einer Krebserkrankung erlauben. Dies kann geschehen über die Einbeziehung histochemischer Marker, wie dies beispielsweise beim Mammakarzinom im Rahmen der Untersuchung der Expression von Östrogenrezeptoren erfolgt. Ein alternativer Weg ist die Suche nach Gendefekten, z. B. somatischen Mutationen in Tumorsuppressorgenen. Neuere Ansätze versuchen den Krankheitsverlauf mit Hilfe von Genexpressionsprofilen vorherzusagen.

Ein weiteres Problem bei der Patientenbehandlung ist die Vorhersage der Wirksamkeit von Medikamenten und spezifischen Ernährungsregimen, der individuell optimalen Dosierung bzw. Therapiedauer, aber auch die Vorhersage des Auftretens schwerwiegender und bedrohlicher Nebenwirkungen. Beispielsweise können genetische Polymorphismen im Gen *UGT1A1* dafür verantwortlich gemacht werden, dass bei einigen Patienten unter Therapie mit Irinotecan schwerwiegende Nebenwirkungen auftreten. Eine vorherige Genanalyse kann solche Patienten vor Therapiebeginn identifizieren und eine Dosisanpassung ermöglichen. Daneben sind Therapien mit in biotechnologischen Verfahren hergestellten Medikamenten teuer und werden zukünftig auf die Patienten beschränkt werden, bei denen eine Wirksamkeit auf Grund der individuellen Disposition wahrscheinlich erscheint. Der vorliegenden Erfindung liegt daher die Aufgabe zu Grunde, ein Mittel bereitzustellen, das eine bessere Prognose des natürlichen Verlaufs einer Erkrankung und das Ansprechen auf jedwede Therapieform erlaubt. Insbesondere soll dieses Mittel auch dazu in der Lage sein, diejenigen Patienten zu erkennen, bei denen spezifische Medikamente wirksam sein werden.

Krebszellen sind gekennzeichnet durch einen Verlust der Kontaktinhibition und unkontrolliertes Zellwachstum. Ausgelöst werden solche Veränderungen spontan oder durch Noxen, sogenannte Kanzerogene, welche das Erbgut schädigen. Zu solchen Noxen gehören viele Chemikalien, Tabakrauch, aber auch UV-Licht. Daneben spielen genetische Faktoren bei der Krebsentstehung eine herausragende Rolle. Kennzeichnend für Krebszellen ist neben ihrem ungehemmten Wachstum auch die Neigung, Tochtergeschwülste (Metastasen) in anderen Organen abzusiedeln. Die Ausbreitung der Metastasen erfolgt regelmäßig über die Blutbahn oder über Lymphgefäße. Krebserkrankungen sind bei einem Großteil der Fälle nicht heilbar und führen zum Tode. Therapeutisch wird versucht, den Ausgangstumor und Metastasen operativ zu entfernen. Daneben können Tumore bestrahlt werden. Mittels so genannter Zytostatika, Antikörper gegen bestimmte Proteine oder Oberflächenmarker oder immunmodulierender Substanzen (Zytokine, Interferone) wird versucht, die sich schnell teilenden Krebszellen abzutöten oder in den programmierten Zelltod (Apoptose) zu überführen.

Es ist von medizinisch überaus hoher Relevanz, Prognosefaktoren für den Verlauf von Krebserkrankungen zu definieren, die Auskunft über das Ansprechen auf bestimmte Therapieformen geben oder die generell prädiktiv für das Auftreten von Metastasen, die Tumorprogression und das Überleben sind. Bislang werden in der Medizin dem Fachmann allgemein bekannte Prognosefaktoren eingesetzt. Hierzu gehören beispielsweise die Größe des Tumors, seine Eindringtiefe in das umgebende Gewebe, organüberschreitendes Wachstum, das Eindringen in Blut- oder Lymphgefäße oder in Lymphknoten, sowie der Differenzierungsgrad der Tumorzellen. Daneben existieren einige relativ unspezifische serologische Marker. Das Verfahren zur Klassifikation der Tumore wird allgemein als Staging" und Grading" bezeichnet. Generell gilt, dass das Vorhandensein von Fernmetastasen und ein geringer Differenzierungsgrad prognostisch sehr ungünstige Parameter darstellen. Dennoch ist es die allgemeine Erfahrung in der Medizin, dass Patienten bei gleichem Tumorstadium drastisch unterschiedliche Krankheitsverläufe aufweisen. Während man bei manchen Patienten eine schnelle Progression der Erkrankung und das Auftreten von Metastasen und Rezidiven beobachtet, kommt die Erkrankung bei anderen Patienten aus unklaren Gründen zum Stillstand. Metastasen können hierbei lokal, regional und fern der Muttergeschwulst auftreten. Dazu ist es notwendig, dass eine hohe Zahl von Geschwulstzellen über den Lymph- oder Blutweg durch einfache Fortschwemmung in Flüssigkeit oder durch Abklatschen in benachbartes Gewebe gelangt. Unter Rezidivneigung versteht man das erneute Auftreten einer Geschwulst nach operativer unvollständiger oder Teilentfernung des Tumors. Hierbei handelt es sich nicht um eine erneute maligne Transformation, sondern um das Nachwachsen eines nicht vollständig entfernten Tumorgewebes. Eine Rezidivbildung ist auch durch Metastasen möglich, die viele Jahre latent bleiben können. Unter Progression versteht man das Wiederauftreten eines Tumors mit höherem Grading (mehr Entdifferenzierung) oder das Neuauftreten von Metastasen.

Ganz offensichtlich gibt es eine Vielzahl individueller, unerkannter biologischer Variablen, die den Verlauf einer Tumorerkrankung unabhängig von Staging und Grading in hohem Maße determinieren. Zu solchen Faktoren gehören genetische Wirtsfaktoren.

Daneben ist es wünschenswert, genetische Marker zu entwickeln, die prädiktiv für das Auftreten von Tumoren sind. Solche Marker erfüllen die Funktion, die betroffenen Individuen frühzeitig weiteren Screeningmaßnahmen (Serologie, Röntgen, Ultraschall, NMR etc.) zuzuführen. Damit können Krebserkrankungen im Frühstadium erkannt und therapeutisch angegangen werden, wobei die Heilungs- und Überlebenschancen bei Tumoren im Frühstadium wesentlich besser sind als bei fortgeschrittenen Tumoren.

Generell können alle Zellen des menschlichen Körpers maligne entarten und zu einer Krebserkrankung führen. Die hier und im Weiteren gemachten Ausführungen beschreiben generelle Mechanismen der Tumorprogression, der Metastasierung und des therapeutischen Ansprechens. Insofern gelten die hier beschriebenen Mechanismen und Ansprüche für alle Tumore des Menschen, beispielsweise für die folgenden Tumoren:
- Tumoren des Urogenitaltrakts: Hier sind zu nennen das Harnblasenkarzinom, das Nierenzellkarzinom, das Prostatakarzinom und das Seminom.
- Tumoren der weiblichen Geschlechtsorgane: Das Mammakarzinom, das Korpuskarzinom, das Ovarialkarzinom, das Zervixkarzinom.
- Tumoren des Gastrointestinaltraktes: Das Mundhöhlenkarzinom, das Ösophaguskarzinom, das Magenkarzinom, das Leberkarzinom, das Gallengangskarzinom, das Pankreaskarzinom, das Kolonkarzinom, das Rektumkarzinom.
- Tumore des Respirationstraktes: das Kehlkopfkarzinom, das Bronchialkarzinom.
- Tumore der Haut: das maligne Melanom, das Basaliom, das T-Zell-Lymphom.
- Tumorerkrankungen des blutbildenden Systems: Hodgkin- und Non-Hodgkin-Lymphome, akute und chronische Leukämien, Plasmozytom, etc.
- Tumorerkrankungen des Gehirns bzw. des Nervengewebes: Glioblastom, Neuroblastom, Medulloblastom, meningeales Sarkom, Astrozytom.
- Weichteiltumore, beispielsweise Sarkome und Kopf-Hals-Tumore.

Die obigen Ausführungen zu genetischen Prognosefaktoren sind nicht beschränkt auf maligne Erkrankungen. Auch in anderen Bereichen sind diese von Bedeutung. Beispielhaft zu nennen sind:
- Herz-Kreislauf-Erkrankungen: Hypertonie, Koronare Herz Krankheit, Arteriosklerose, Herzinsuffizienz, Kardiomyopathie, etc.
- Stoffwechselerkrankungen: Diabetes mellitus, Gicht, Fettstoffwechselstörungen, etc.
- Entzündungen und Infektionen: Rheumatische Erkrankungen, parasitäre Erkrankungen, Pilzinfektionen, etc.

Prenylierung ist eine posttranslationale Modifikation von Proteinen durch kovalente Bindung von speziellen Lipiden (Farnesyl- oder Geranlygeranyl-Isoprenoide). Die Prenylierung ermöglicht Proteinen ohne eigene proteinäre Membrandomäne die Interaktion mit der Membran und konnte bereits bei über 100 Proteinen als essentielle Modifikation für eine korrekte Funktion nachgewiesen werden. Viele dieser Proteine nehmen entscheidende Aufgaben in der Signaltransduktion der Zelle wahr. Bisher wurden drei Enzyme identifiziert, die in zwei funktionelle Klassen unterteilt werden können. Die beiden Enzyme Farnesyltransferase (FTase) und Geranylgeranyltransferase-I (GGTase-I) bilden die Unterklasse der CAAX-Prenyltransferasen, die Geranylgeranyltransferase-II bildet eine zweite eigenständige Unterklasse. Die CAAX-Prenyltransferasen haben beide als Erkennungsmotiv die sogenannte CAAX-Box und führen beide zu einer Prenylierung am Cysteinresiduum nahe des C-terminalen Endes der Proteine. Welches der beiden Enzyme das jeweilige Protein prozessiert, ist von der C-terminalen Aminosäure abhängig. Spezifische Aminosäuren für die Prenylierung durch die FTase sind z. B. Serin, Methionin, Alanin und Glutamin. FTase und GGTaseI teilen sich eine gemeinsame α-Untereinheit und unterscheiden sich nur in der jeweiligen β-Untereinheit voneinander.

Die β-Untereinheit der FTase ist 46 kDa groß und besteht aus 14 α-Helices, die eine Fassstruktur bilden, in deren Inneren sich die enzymatisch aktive Region befindet. Die β-Untereinheit ist sowohl für die Bindung des Farnesyldiphophats, als auch für die Bindung der zu modifizierenden Proteine zuständig (Lane, K. T., Beese, L. S., J Lipid Res 47: 681-699 (2006)).

Viele Proteine müssen für eine korrekte Funktion prenyliert werden. Eines der ersten identifizierten Substrate der Farnesyltransferase ist die Familie der Ras-Proteine. Diese Gruppe von Proteinen, die essentiell ist für die transmembranäre Signaltransduktion, zählt gleichzeitig zu den ersten identifizierten Onkogenen. Aktivierende Mutationen in Mitgliedern der Ras-Familie finden sich bei ungefähr 20 % aller malignen humanen Tumore. Zusätzlich finden sich bei vielen anderen Tumoren Veränderungen in Faktoren upstream von Ras, die zur Überexpression von Ras-Proteinen führen. Sowohl die überexprimierten als auch die mutierten Ras-Proteine sind auf die posttranslationale Modifikation der Prenylierung angewiesen und können auch ihre pathologische Funktion im Rahmen der Onkogenese nur nach dieser Modifikation wahrnehmen. Außer der zuerst identifizierten Ras-Familie sind mittlerweile weitere wichtige Proteine der Onkogenese identifiziert worden, die ebenfalls auf die Farnesylierung angewiesen sind (Konstantinopoulos, P.A. et al., Nat. Rev. Drug Discov. 6:541-555 (2007)).

Auch bei anderen Erkrankungen spielen farnesylierte Proteine eine entscheidende Rolle. Für das Herz-Kreislauf-System konnte gezeigt werden, dass Hras, ein Mitglied der Ras-Familie, von großer Bedeutung ist für die Hypertrophie des Herzens (Thorburn, A. et al., J. Biol. Chem. 268:224-2249 (1993)) und auch die Rezeptordownregulation adrenerger β-Rezeptoren wird durch farnesylierte Proteine beeinflusst (Chuang, T. T. et al., Trends Pharmacol. Sci. 17:416-421 (1996)).

Auch akute und chronische Entzündungsreaktionen können nur mit Hilfe von farnesylierten Proteinen ablaufen (Abeles, A. M. et al., Arthritis Rheum. 56:2840-2853 (2007))

Bei Patienten mit Progeroid Syndromen hat man die Farnesylierung eines mutierten Proteins als eigentlichen krank machenden Schritt identifizieren können (Young, S. G. et al., J. Biol. Chem. 281:39741-39745 (2006)).

Auch bei der Malaria und anderen Infektionskrankheiten spielt die Farnesyltransferase der Parasiten eine wichtige Rolle. (Eastman, R. T. et al., J. Lipid Res. 47:233-240 (2006)).

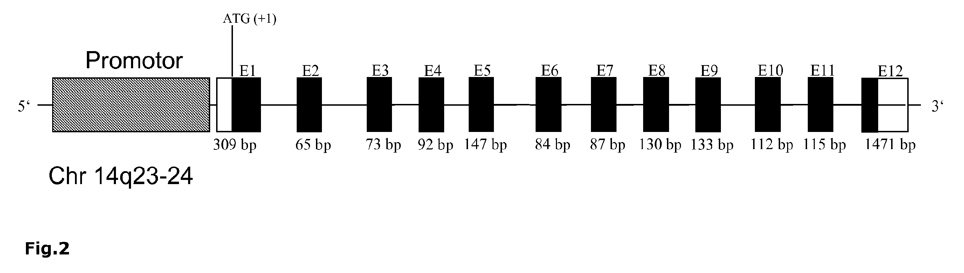

Das Gen *FNTB,* das für die β-Untereinheit der Farnesyltransferase kodiert, ist auf Chromosom 14q23-q24 lokalisiert (Gene bank accession number NM_002028). Es besteht aus 12 Exons, eine schematische Darstellung der Genstruktur findet sich in Fig. 2. Genauere Untersuchungen, z. B. des Genpromotors oder der 3'-untranslatierten Region, sind bisher nicht erfolgt.

### Gegenstand der Erfindung

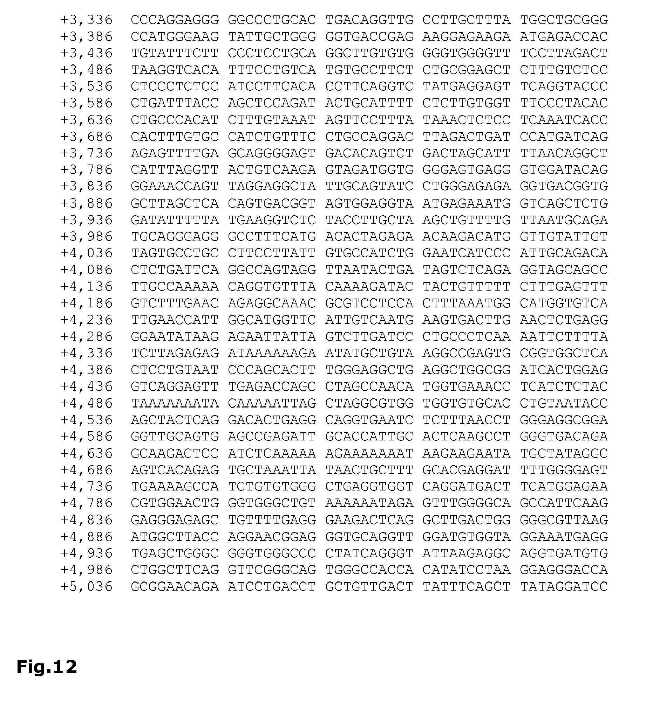

Es wurde nunmehr gefunden, dass bestimmte funktionsverändernde genomische Polymorphismen in der Promotorregion des Gens *FNTB* zur Änderung der Proteinexpression führen und dass diese geeignet sind, generell Krankheitsrisiken und -verläufe sowie das Ansprechen auf Pharmaka und Krebstherapien, insbesondere Farnesyltransferase-Inhibitoren und Statine, und Nebenwirkungen vorherzusagen. Die Polymorphismen sind weiterhin zum Auffinden und/oder Validieren weiterer Polymorphismen bzw. Haplotypen im Gen *FNTB* geeignet. Die Erfindung betrifft somit
(1) ein *In-vitro* Verfahren zur Vorhersage von Krankheitsrisiken, Krankheitsverläufen, Arzneimittelrisiken und zum Auffinden von Drug Targets, das das Durchmustern einer biologischen Probe nach einer oder mehreren Genveränderung(en) in der Promotorregion des Gens *FNTB,* das für die β-Untereinheit des Proteins Farnesyltransferase kodiert, auf dem Humanchromosom 14q23-24 umfasst;
(2) eine bevorzugte Ausführungsform des Verfahren (1), wobei die biologische Probe nach einem oder mehreren der Polymorphismen -609, -179 oder -173 der in Fig. 12 gezeigten Sequenz in der Promotorregion des Gens *FNTB* durchmustert wird, wobei bei diesen Polymorphismen bei Position -609 ein Guanin durch ein Cytosin, bei Position -179 ein Thymin durch ein Adenin substituiert ist und bei Position -173 ein Guanin deletiert ist;
(3) ein Verfahren zum Auffinden relevanter Polymorphismen und Haplotypen, im Gen *FNTB* und in dazu benachbarten Genen umfassend die Quantifizierung der Expression von FNTB im Zusammenhang mit dem vorstehend unter (1) und (2) beschriebenen Polymorphismus und Abgleich mit der Expression von anderen Polymorphismen der *FNTB,* und
(4) ein Kit zur Durchführung des Verfahrens nach Anspruch (1) oder (2) umfassend Primer und/oder Sonden, die zur Detektion der Polymorphismen -609, -179 und/oder -173 der in Fig. 12 gezeigten Sequenz in der Promotorregion des Gens *FNTB* geeignet sind.

### Kurzbeschreibung der Figuren

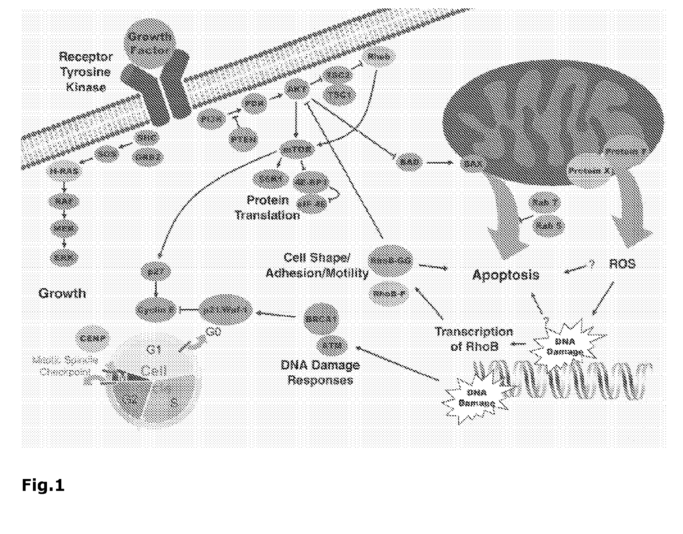

Fig. 1: Schematische Darstellung einiger Signalkaskaden, an denen farnesylierte Proteine beteiligt sind (nach Pan, J. et al.; Cancer Res. 65(20): 9109-12 (2005)).
Fig. 2: Exon/Intron-Struktur des humanen Gens *FNTB* (nicht maßstabsgetreu).
Fig. 3: Darstellung der Promotor-Sequenz von *FNTB* mit flankierenden Sequenzen (SEQ ID NO:2). An den Positionen -609, -179 und -173 sind die hier beschriebenen Polymorphismen fett dargestellt.
Fig. 4: EMSA des SNPs -609 mit HEK 293 Zellkernextrakten (A) und KARPAS 422 Zellkernextrakten (B).
Fig. 5: EMSA der SNPs -173 und -179 mit HEK 293 Zellkernextrakten.
Fig. 6: EMSA des SNPs -173 mit HEK 293 Zellkernextrakten (A) und KARPAS 422 Zellkernextrakten (B).
Fig. 7: Genotyp-abhängige Darstellung der *FNTB*-Expression relativ zu β-Actin in Leukozyten gesunder Probanden und Nierenzellkarzinomgewebe.
Fig. 8: -609 Genotyp-abhängige Darstellung des Überlebens als Kaplan-Meier-Kurven bei Patienten mit Nierenzellkarzinom und einem Follow-up von 240 Monaten.
Fig. 9: Kaplan-Meier-Analyse zum Überleben von Patienten mit Plasmozytom abhängig vom Genotyp des *FNTB*-Promotor-Polymorphismus -609.
Fig. 10: Kaplan-Meier-Analyse zum Überleben von Patientinnen mit Mammakarzinom abhängig vom Genotyp des *FNTB*-Promotor-Polymorphismus -173 und einem Follow-up von 60 Monaten.
Fig. 11: Kaplan-Meier-Analyse zum Überleben von Patienten mit malignem Melanom abhängig vom Genotyp des *FNTB*-Promotor-Polymorphismus -173.
Fig. 12: Putative Promotorregion für das Gen FNTB (SEQ ID No:1). Die Nummerierung erfolgt ausgehend vom A des Startkodons ATG, als +1, und ausgehend vom C 5' von diesem A als -1. Kleinbuchstaben: genomische Sequenz vor dem Exon1, kursive Kleinbuchstaben: 5'UTR des Exon 1; fette Kleinbuchstaben: Polymorphismen; kursive Großbuchstaben: kodierende Sequenz, Großbuchstaben: genomische Sequenz, Intron 1.

### Detaillierte Beschreibung der Erfindung

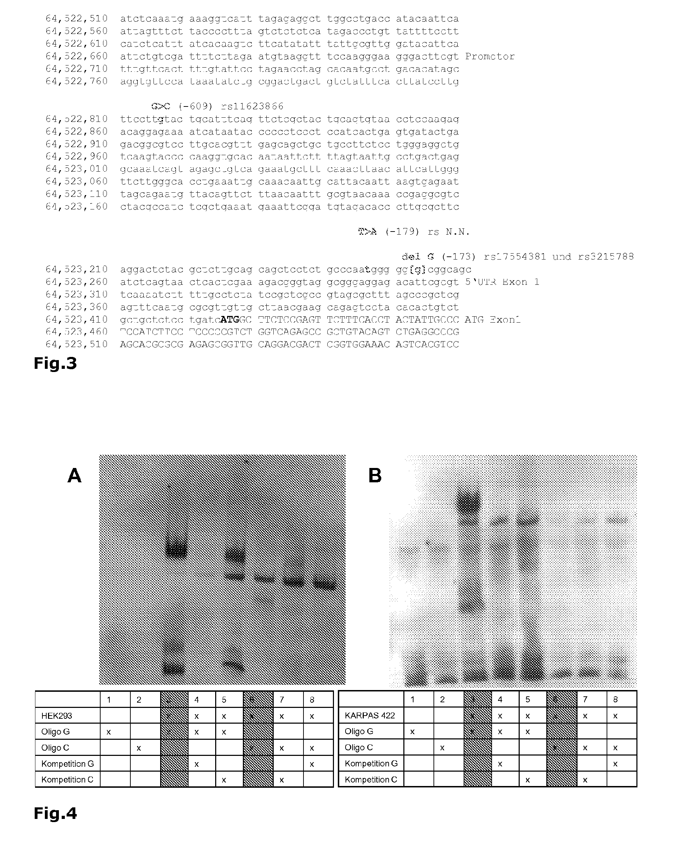

Die vorliegende Erfindung beruht auf der Bereitstellung der in der Promotorregion von *FNTB* befindlichen Polymorphismen, die durch systematische Sequenzierung von DNAs von Menschen gefunden wurde. Hierzu wurden Gensequenzen, die zum Promotor und zur 5'UTR des Exon 1 gehören, aber nicht translatiert werden, mittels PCR-Reaktionen amplifiziert und gemäß der Methode nach Sanger sequenziert. Die dazu erforderlichen Verfahren, z. B. das Ableiten von für die PCR-Reaktion erforderlichen Primerpaaren und die Auswahl von Sequenzier-Primern, sind dem Fachmann geläufig. Hierbei wurden drei Polymorphismen detektiert, wobei in der Promotorregion an Position -609 ein Basenaustausch von Guanin zu Cytosin vorliegt (rs11623866), an Position -179 ein Basenaustausch von Thymin zu Adenin (rs N.N.) und an Position -173 eine Deletion eines Guanin (rs17554381 und rs3215788). In Fig. 3 sind diese Polymorphismus mit ihren flankierenden Sequenzen dargestellt. Die Nummerierung der Position erfolgt in der Weise, dass dem Nukleotid vor dem A des Startkodons ATG die Nummer -1 zugeordnet wird.

Der Nachweis dieser SNPs gemäß Aspekt (1) und (2) der vorliegenden Erfindung kann mit beliebigen, dem Fachmann geläufigen Verfahren nachgewiesen werden. Geeignete Verfahren sind z. B. direkte Sequenzierung, PCR mit nachfolgender Restriktionsanalyse, reverse Hybridisierung, Dot-blot- oder slot-blot-Verfahren, Massenspektrometrie, Taqman®- oder Light-Cycler®-Technologie, Pyrosequencing®, Invader®-Technologie, Luminex-Verfahren, DNA-Chips etc.

Der Nachweis des Polymorphismus in genomischer DNA mittels direkter Sequenzierung (z. B. nach der Sanger-Methode, der Pyrosequenzierung, der Maxam und Gilbert-Methode, der Solexa-Technologie oder Sequenzierung durch Ligation) kann mit Hilfe von Primerpaaren und/oder zusätzlichen Sequenzierprimern erfolgen, die wenigstens 10 zusammenhängende Nukleotide aus den Nukleotiden -5000 und +5000, vorzugsweise aus den Nukleotiden -2609 bis +1391 für den SNP -609, -2179 bis +1821 für den SNP -179 und -2173 bis +1827 für den SNP -173 der in Fig. 12 gezeigten Sequenz, wobei die Primerpaare den jeweiligen Polymorphismus einschließen und der Nachweis auch gemeinsam erfolgen kann, umfassen. Als Beispiel für ein solches Primerpaar dienen die angegebenen in Beispiel 1 verwendeten Primersequenzen.

Der Nachweis des Polymorphismus in genomischer DNA mittels MALDI-TOF-MS basierter DNA-Analytik kann mit Hilfe von Primerpaaren erfolgen, die wenigstens 10 zusammenhängende Nukleotide aus den Nukleotiden -5000 und +5000, vorzugsweise aus den Nukleotiden -2609 bis +1391 für den SNP - 609, -2179 bis +1821 für den SNP -179 und -2173 bis +1827 für den SNP - 173 der in Fig. 12 gezeigten Sequenz, wobei die Primerpaare den jeweiligen Polymorphismus einschließen und der Nachweis auch gemeinsam erfolgen kann, umfassen. Nach anschließender Polymerasekettenreaktion (PCR) kann durch Extension eines Primers, der aus wenigstens 10 zusammenhängenden Nukleotiden umfasst, welche in 5'-Richtung oder in 3'-Richtung ausgehen von den jeweiligen Polymorphismus-Positionen in der in Fig. 12 gezeigten Sequenz, der Polymorphismus über die unterschiedlichen Nukleotidmassen genotypisiert werden.

Der Nachweis der Polymorphismen in genomischer DNA mittels Restriktionsfragment-Längenpolymorphismus (RFLP) erfolgt nach einer PCR mit Primern, die wenigstens 10 zusammenhängende Nukleotide aus den Nukleotiden -5000 und +5000, vorzugsweise aus den Nukleotiden -2609 bis +1391 für den SNP -609, -2179 bis +1821 für den SNP -179 und -2173 bis +1827 für den SNP - 173 der in Fig. 12 gezeigten Sequenz, wobei die Primerpaare den jeweiligen Polymorphismus einschließen und der Nachweis auch gemeinsam erfolgen kann, umfassen. Zudem ist es möglich, durch einen gezielten Basenaustausch in einem Primer (Mutageneseprimer) eine Restriktionsschnittstelle zu generieren. Als Beispiel dafür dienen die im Beispiel 1 angegebenen Primer zu den Polymorphismen -173 und -179 in Fig. 12. Diese Vorgehensweise kann für beliebige weitere Restriktionsenzyme angewandt werden.

Der Nachweis des Polymorphismus in genomischer DNA mittels allelspezifischer PCR kann mit Hilfe von Primerpaaren erfolgen, die wenigstens 10 zusammenhängende Nukleotide aus den Nukleotiden -5000 und +5000, vorzugsweise aus den Nukleotiden -2609 bis +1391 für den SNP -609, -2179 bis +1821 für den SNP -179 und -2173 bis +1827 für den SNP -173 der in Fig. 12 gezeigten Sequenz, wobei die Primerpaare den jeweiligen Polymorphismus einschließen und der Nachweis auch gemeinsam erfolgen kann, umfassen. Davon muss jeweils ein Primer spezifisch für die jeweiligen Allele an den Position -609, -179 und -173 vorliegen.

Der Nachweis des Polymorphismus in genomischer DNA mittels Hochleistungsflüssigkeitschromatographie (HPLC) kann mit Hilfe von Primerpaaren, die wenigstens 10 zusammenhängende Nukleotide aus den Nukleotiden -5000 und +5000, vorzugsweise aus den Nukleotiden -2609 bis +1391 für den SNP -609, -2179 bis +1821 für den SNP -179 und -2173 bis +1827 für den SNP - 173 der in Fig. 12 gezeigten Sequenz, wobei die Primerpaare den jeweiligen Polymorphismus einschließen und der Nachweis auch gemeinsam erfolgen kann, umfassen, anschließender PCR und dem Aufschmelzen der Amplifikate über die Bildung von Heteroduplexen erfolgen.

Der Nachweis des Polymorphismus in genomischer DNA mittels allelspezifischer Hybridisierung (z. B. Taqman^{®}- oder Light-Cycler^{®}-Technologie) kann mit Hilfe von Primerpaaren, die wenigstens 10 zusammenhängende Nukleotide, aus den Nukleotiden -5000 und +5000, vorzugsweise aus den Nukleotiden -2609 bis +1391 für den SNP -609, -2179 bis +1821 für den SNP -179 und - 2173 bis +1827 für den SNP -173 der in Fig. 12 gezeigten Sequenz, wobei die

Primerpaare den jeweiligen Polymorphismus einschließen und der Nachweis auch gemeinsam erfolgen kann, umfassen, anschließender PCR und dem Verwenden von allelspezifischen Sonden erfolgen. Auch ohne vorherige PCR-Amplifikation können allelspezifische Sonden zur Detektion des Polymorphismus eingesetzt werden (z. B. DNA- oder RNA/cDNA-Micro Arrays, DNA- oder RNA/cDNA-Blotting-Verfahren, Luminex^{®}- oder Invader^{®}-Technologie). Diese Sonden umfassen eine Sequenz, die wenigstens 10 zusammenhängende Nukleotide aus den Nukleotiden -5000 und +5000, vorzugsweise aus den Nukleotiden -2609 bis +1391 für den SNP -609, -2179 bis +1821 für den SNP -179 und -2173 bis +1827 für den SNP -173 der in Fig. 12 gezeigten Sequenz aufweist, wobei sie spezifisch für das jeweilige Allel der Polymorphismen an genomischer Position -609, -179 und/oder -173 sind.

Es ist dabei bevorzugt, dass die Primer wenigstens 15, vorzugsweise 15 bis 30 zusammenhängende Nukleotide der Sequenzen von Fig. 12 aufweisen und vorzugsweise die Sequenz von SEQ ID NOs:3 bis 9, Nukleotide -2609 bis +1391 für den SNP -609, -2179 bis +1821 für den SNP -179 und -2173 bis +1827 für den SNP -173 der in Fig. 12 gezeigten Sequenz aufweisen. Ebenfalls bevorzugt ist, dass die Sonden wenigstens 20 zusammenhängende Nukleotide der Sequenzen von Fig. 12 aufweisen.

Das Verfahren gemäß Aspekt (1) und (2) der Erfindung ist insbesondere das zur Voraussage von Risiken oder dem Verlauf einer Erkrankung, einschließlich Nierenzellkarzinom, Plasmozytom, Mammakarzinom und malignes Melanom geeignet ist, wobei bei -609 der Genotyp GG, bei -179 der Genotyp TT und bei -173 der Genotyp 5G/5G die beste Überlebensprognose zeigt. In einem weiteren Aspekt ist das Verfahren zum Auffinden und Evaluieren von Krebstherapeutika geeignet.

Gemäß Aspekt (3) der Erfindung können die detektierten Polymorphismen dazu benutzt werden, um weitere relevante genomische Genveränderungen in *FNTB* oder benachbarten Genen zu identifizieren und zu validieren, die z. B. mit Genotypen im Gen *FNTB* im Kopplungsungleichgewicht stehen. Dies können auch Gene sein, die ebenfalls auf Chromosom 14 liegen, aber in großer Entfernung vom Gen *FNTB.* Hierzu wird folgendermaßen vorgegangen:
1. Für bestimmte Phänotypen (zelluläre Eigenschaften, Krankheitszustände, Krankheitsverläufe, Arzneimittelansprechen usw.) wird zunächst eine Assoziation mit den Genotypen des *FNTB*-Polymorphismus hergestellt.
2. Für neu detektierte Genveränderungen in *FNTB* oder benachbarten Genen wird untersucht, ob bereits bestehende Assoziationen unter Verwendung oben beschriebener Genotypen verstärkt oder abgeschwächt werden.

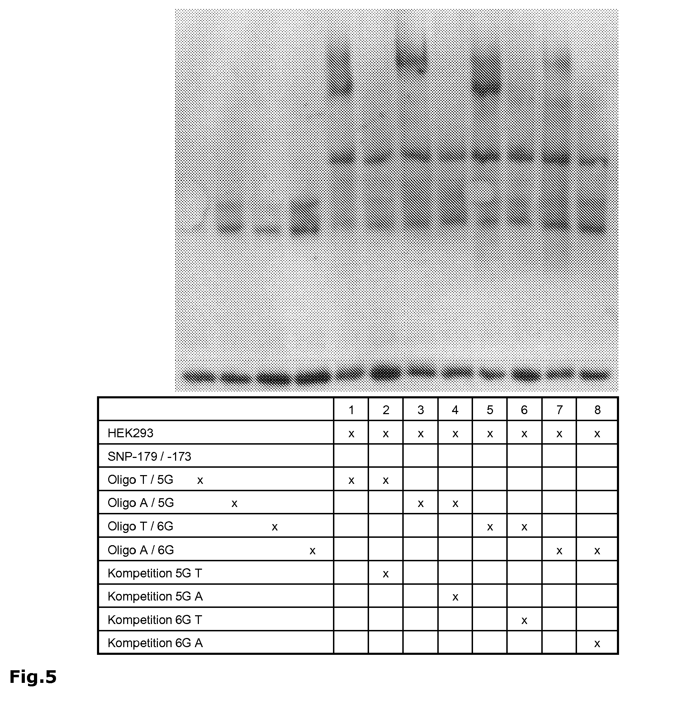

Es wurde untersucht, welche funktionellen Änderungen den Promotor-Polymorphismen im Gen *FNTB* zuzuordnen sind. Hierzu wurde zunächst mittels eines Computer-Programms, dann mit Hilfe von Electrophoretic mobility shift assay (EMSA) untersucht, ob die Basensubstitutionen die Bindung von Transkriptionsfaktoren oder anderen regulatorischen Proteinen verändern kann. Diese Proteine binden an spezifische Konsensus-Sequenzen in der Promotorregion von Genen und können dadurch die Aktivität eines Promotors und damit die mRNA-Expression beeinflussen. Wie in den Fig. 4 - 6 dargestellt, zeigen alle drei Polymorphismen eine allelspezifische Bindung sowohl in HEK 293 (humane embryonale Nierenzellen) als auch in KARPAS 422 (Lymphom-Zellinie).

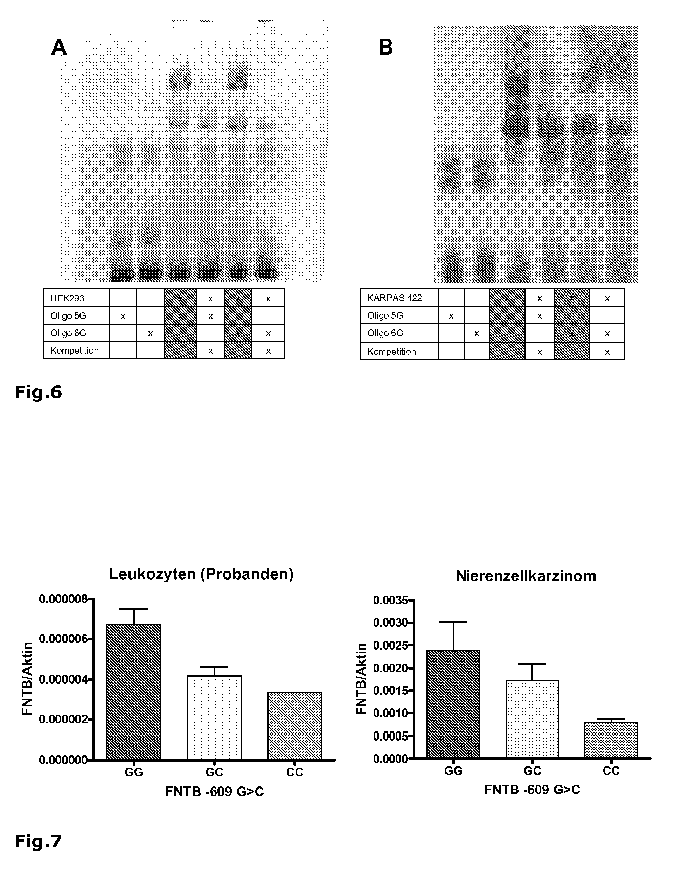

Des Weiteren wurde untersucht, ob die Expression von *FNTB* auf mRNA-Ebene Genotyp-abhängig unterschiedlich ist. Hierfür wurde mRNA aus menschlichem Operationsgewebe bei Nierenzelloperationen und aus Blut von gesunden Probanden gewonnen und mittels reverser Transkriptase in cDNA umgeschrieben. Das Verfahren ist dem Fachmann geläufig. Nachfolgend wurde das Expressionsniveau mittels Realtime-PCR (SYBRGreen-Verfahren) bestimmt und mit dem Expressionsniveau des Housekeeping-Gens β-*Actin* abgeglichen. Die Ergebnisse sind in der Fig. 7 dargestellt. Der GG-Genotyp des -609 SNPs weist eine höhere mRNA-Expression auf als der CC-Genotyp. Die Werte des heterozygoten Genotyps liegen dazwischen, was für einen Gen-Dosis-Effekt spricht.

Damit wurde nachgewiesen, dass es im Gen *FNTB* Genveränderungen gibt, die eine Expressionsänderung von FNTB in benignem und malignem Gewebe bewirken. Dieses können die oben beschriebenen Promotor-Polymorphismen sein oder Polymorphismen, die mit diesen SNPs im Kopplungsungleichgewicht stehen. Bestandteil der hier beschriebenen Erfindung ist damit auch, die Expression von *FNTB* zu quantifizieren, mit bekannten Polymorphismen der Farnesyltransferase zu assoziieren und neue, noch besser geeignete Polymorphismen zu entdecken und zu validieren.

Die hier gezeigten Befunde einer Genotyp-abhängigen Expression von *FNTB* in humanem Gewebe ist bedeutend, da eine sehr hohe Aktivität von FNTB auch die verstärkte Farnesylierung und damit Aktivierung von z. B. Ras-Proteinen ermöglicht, wodurch benignes oder malignes Zellwachstum begünstigt werden und sich negativ z. B. auf die Tumorprogression auswirken. Zudem kann sich diese Genotyp-abhängige Genexpression von *FNTB* auch auf das Ansprechen auf eine Therapie mit Farnesyltransferase-Inhibitoren auswirken. Es ist zu erwarten, dass eine niedrige Genexpression prädisponiert durch einen bestimmten Genotyp, z. B. der CC-Genotyp des -609 Polymorphismus, stärker auf Farnesyltransferase-Inhibitoren anspricht als die anderen Genotypen. Damit können Genveränderungen im Gen *FNTB* dazu verwendet werden, das Ansprechen auf eine Farnesyltransferase-Inhibitoren Therapie vorherzusagen, um zum Beispiel Responder versus Non-Responder zu diskriminieren oder um eine genotypabhängige Dosierung vorzunehmen. Als typische Vertreter solcher Inhibitoren können die Substanzen Tipifarnib und Lonafarnib genannt werden. Diese Genveränderungen können auch für eine Dosisfindung eingesetzt werden bzw. für die Vorhersage des Auftretens unerwünschter Arzneimittelwirkungen. Solche Therapien können im weitesten Sinne medikamentös erfolgen, d. h. durch Zuführung von Substanzen in den Körper oder diese Therapeutika können physikalisch wirksam sein.

Wir erwarten somit die Beeinflussung von Krankheitsverläufen, insbesondere bei von der Farnesyltransferase direkt oder indirekt beeinflussten Erkrankungen sowie ein geändertes Ansprechen auf Substanzen, die die Regulationskaskade von FNTB beeinflussen oder Substanzen, die direkt FNTB inhibieren.

Aufgrund der Schlüsselfunktion der Farnesyltransferase für die Proteine der verschiedenen Signalwege der Zelle ist es ein wesentlicher Bestandteil der Erfindung, dass unter Verwendung von Genveränderungen in *FNTB* generell Erkrankungsrisiken und Krankheitsverläufe vorhergesagt werden können.

Für Krebserkrankungen bedeutet das z. B., dass die Multistep-Entwicklung von Krebs die Akkumulation genetischer Veränderungen widerspiegelt, die zur Transformation von normalen Zellen zu Krebszellen und von normalem Gewebe zu benignen und evtl. malignen, invasiven Tumoren führen. Die Ansammlung von Alterationen beschleunigt die Tumorgenese und kann auch die Therapie beeinflussen. Es wird allerdings auch deutlich, dass gestörte Mechanismen der Zellzyklusregulation der Grund für die erhöhte genomische Instabilität von Tumoren sind (Hoeijmaker, J. H., Nature 411:366-74 (2001)). Da die Farnesylierung nicht nur bei Ras-Proteinen sondern auch bei vielen anderen Proteinen wie z. B. bei den promitotischen Zentromer-Proteinen CENP-E und CENP-F eine nötige posttranslationale Modifikation darstellt, ist unmittelbar zu erwarten, dass der Verlauf vielfältiger und ganz unterschiedlicher Tumorerkrankungen bei einer genetisch determinierten, geänderten Aktivierbarkeit der Farnesyltransferase beeinflusst wird (Crespo, N. C. et al., J. Biol. Chem. 276:16161-16167 (2001); Ashar, H. R. et al., J. Biol. Chem. 275:20451-30457 (2000)). Dies bedeutet, dass durch Expressionsänderungen von Proteinen, die in allen menschlichen Körperzellen exprimiert werden und die über posttranslationale Modifikationen Proteine der Zellteilung regeln, Zellfunktionen bestimmt werden, die alle physiologischen und pathophysiologischen Vorgänge entscheidend beeinflussen oder zumindest modulieren. Daneben werden auch Antworten auf Pharmaka in besonderer Weise beeinflusst. Hiervon sind erwünschte, aber auch unerwünschte Arzneimittelwirkungen betroffen.

Als Erkrankungen, die mit einer Genveränderung in *FNTB* einhergehen, können genannt werden:
1. benigne Neoplasien jedes Ursprungsgewebes
2. maligne Neoplasien jedes Ursprungsgewebes
3. Herz-Kreislauf-Erkrankungen
4. Stoffwechsel-Erkrankungen
5. Entzündungen und Infektionen

Ein wesentlicher Bestandteil der vorliegenden Erfindung ist die Bereitstellung diagnostisch relevanter Genveränderungen im Gen *FNTB* als Prognosefaktor für Erkrankungen des Menschen. Naturgemäß können hierbei nicht alle Erkrankungen beschrieben werden.

Da die essentiellen Funktionen der Farnesyltransferase bekannt sind, können Genveränderungen im Gen *FNTB* das Risiko für Erkrankungen erhöhen bzw. Krankheitsverläufe beeinflussen. Es ist generell nicht möglich, alle Erkrankungen des Menschen und deren Verläufe zu untersuchen. Es wurde jedoch in den nachfolgenden Beispielen exemplarisch für vier unterschiedliche Malignome, nämlich Nierenzellkarzinom, Plasmozytom, Mammakarzinom und malignes Melanom gezeigt, dass eine Prognose möglich ist. Diese Daten belegen eindeutig die Verwendbarkeit der Genveränderungen im Gen *FNTB* für den hier beschriebenen Zweck. Diese Erkrankungen stehen *a priori* in keinerlei Zusammenhang.

Die Wirksamkeit von Arzneimitteln und/oder das Auftreten unerwünschter Nebenwirkungen wird neben den spezifischen Stoffeigenschaften der chemisch definierten Produkte durch eine Reihe von Parametern definiert. Zwei wichtige Parameter, die erzielbare Plasmakonzentration und die Plasmahalbwertszeit, bestimmen in hohem Maß die Wirksamkeit oder Unwirksamkeit von Pharmaka oder das Auftreten unerwünschter Wirkungen. Die Plasmahalbwertszeit wird unter anderem dadurch bestimmt, mit welcher Geschwindigkeit bestimmte Pharmaka in der Leber oder anderen Körperorganen zu wirksamen oder unwirksamen Metaboliten verstoffwechselt und mit welcher Geschwindigkeit sie aus dem Körper ausgeschieden werden, wobei die Ausscheidung über die Nieren, über die Atemluft, über den Schweiß, über die Spermaflüssigkeit, über den Stuhl oder über andere Körpersekrete erfolgen kann. Daneben wird die Wirksamkeit bei oraler Gabe durch den sogenannte "first-pass-Effekt" limitiert, da nach Resorption von Pharmaka über den Darm ein bestimmter Anteil in der Leber zu unwirksamen Metaboliten verstoffwechselt wird.

Mutationen oder Polymorphismen in Genen metabolisierender Enzyme können die Aktivität derselben in der Weise verändern, dass deren Aminosäurezusammensetzung verändert wird, wodurch die Affinität zum zu metabolisierenden Substrat erhöht oder erniedrigt wird und damit der Metabolismus beschleunigt oder verlangsamt sein kann. In ähnlicher Weise können Mutationen oder Polymorphismen in Transportproteinen die Aminosäurezusammensetzung in der Weise verändern, dass der Transport und damit die Ausscheidung aus dem Körper beschleunigt oder verlangsamt wird.

Zur Auswahl der für einen Patienten optimal geeigneten Substanz, der optimalen Dosierung, der optimalen Darreichungsform und zur Vermeidung unerwünschter, z. T. gesundheitsschädlicher oder tödlicher Nebenwirkungen ist die Kenntnis von genetischen Polymorphismen oder von Mutation, die zur Änderung der Genprodukte führen, von herausragender Bedeutung.

Aufgrund der großen Bedeutung der Farnesylierung für Onkoprotoeine wird die Inhibierung der Farnesyltransferase als viel versprechende Therapiestrategie angesehen. In vielen verschiedenen Tumorarten stellen diese Inhibitoren entweder einen Teil einer Multichemotherapie dar oder werden als sogenannte First-Line-Therapie als einziges Medikament verabreicht (Basso A. et al., J. Lipid Res. 47: 15-31 (2006)).

Als typische Inhibitoren der Farnesyltransferase können Tipifarnib ((R)-6-amino[(4-chlorophenyl)(1-methyl-1H-imidazol-5-yl)methy I]-4-(3-chlorophenyl)-1-methyl-2(1H)-quinolinone), Lonafarnib (4-[2-[4-(3,10-Dibromo-8-chloro-6,11-dihydro-5H-benzo[5,6]cyclohepta[1,2-b]pyridin-11-yl)-1-piperidinyl]-2-oxoethyl]-1-piperidinecarboxamide), BMS-214662, L778123 und ABT-100 genannt werden.

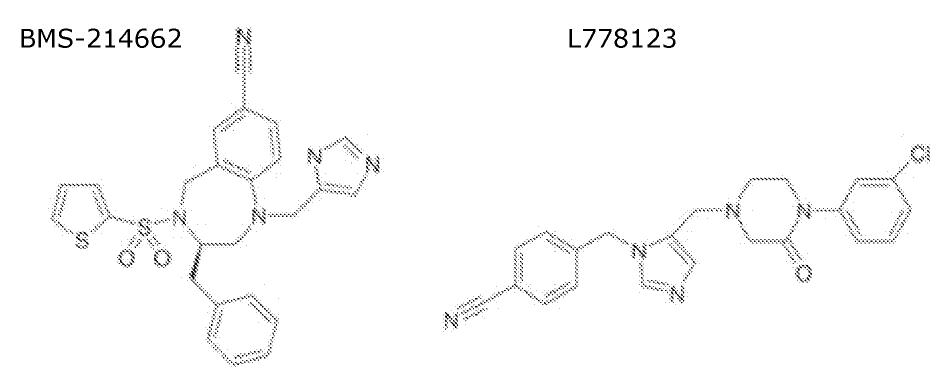

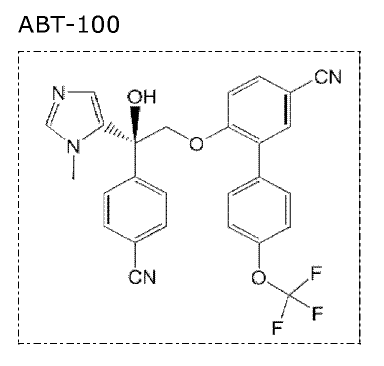

Interessanterweise sind auch Pilze und unterschiedlichste Parasiten sensitiv für FTIs, die auch die menschliche FTase hemmen können. Sie zeigen dabei ein relativ günstiges Nebenwirkungsprofil und werden als zukunftsweisende Therapeutika unter anderem bei Malaria, der Chagas-Krankheit oder bestimmten Candida-Infektionen angesehen (Eastman, R. T. et al., J. Lipid Res. 47:233-240 (2006)). Zusätzlich hierzu sind die FTIs auf Zellkultur und tierexperimenteller Ebene sehr erfolgreich z. B. bei Progeroid-Syndromen eingesetzt worden (Yang, S. H et al., J. Clin. Invest. 116:2115-2121 (2006)).

Es konnte des Weiteren gezeigt werden, dass ein mögliches Einsatzgebiet der FTIs die Entzündungshemmung und Behandlung von akuten Allograft-Abstoßungen ist (Xue, X. et al., JPET 317: 53-60 (2006); Si, M.S et al., J. Heart Lung Transplant 24: 1403-1409 (2005)).

Auch Kollagen-induzierte Arthritis kann mit FTIs behandelt werden (Na, H. J. et al., J. Immunol. 173:1276-1283 (2004)).

Zuletzt sind die Wirkungen der FTIs auf das Herz-Kreislauf-System zu nennen, hier kann sowohl die Entstehung einer Arteriosklerose, als auch die Hypertrophie von Kardiomyozyten verhindert werden (Sugita, M. et al., Arterioscler. Thromb. Vasc. Biol. 27:1390-1395 (2007); Calderone, A. et al., J. Mol. Cell Cardiol. 32:1127-1140 (2000)).

Das Prinzip wird daher an ausgewählten Beispielen erläutert, welche die generelle Verwendbarkeit demonstrieren, die Erfindung jedoch nicht einschränken.

### Beispiele:

### Beispiel 1. Nachweis der Polymorphismen

Die Polymorphismen wurden durch direkte Sequenzierung der putativen Promotorregion von FNTB detektiert. Dazu wurden folgende Primer verwendet:
5'- ATACTGTTCTTCTGGATGACTCCTCGT -3' (SEQ ID NO:8)
5'- TGTTTCCACCGAGTCGTCCTG -3' (SEQ ID NO:9)

Die PCR-Reaktion wurde in einem Ansatz, der einen kommerziellen PCR-Mastermix (2xTaq MasterMIx, Amplicon) enthielt, durchgeführt. Es wurden je 10 pM Primer und 50 ng genomische DNA eingesetzt. Die PCR-Bedingungen waren wie folgt:
95°C 5 min
95°C 30 s ┐
61°C 40 s ∥ 38x
72°C 45 s ┘
72°C 10 min

Nach Aufreinigung des 1078bp PCR-Produktes mittels eines kommerziellen Kits (QIAquick PCR Purification Kit, Qiagen) wurde die Sequenzierung von der Firma Eurofins Medigenomix GmbH (Martinsried) durchgeführt. Die Polymorphismen wurden mittels Restriktionsfragmentlängen-Untersuchung in Patienten und Kontrollen genotypisiert. Folgende Bedingungen kamen zum Einsatz: Polymorphismus -609:
5'- GCGGACTGACTGTCTATTT -3' (SEQ ID NO:3)
5'- GACGCCGTCTCTCAGTATCA -3' (SEQ ID NO:4)

PCR-Produkt 140bp, Verdau mit dem Restriktionsenzym RsaI, es wird nur das G-Allel geschnitten. Dadurch ergeben sich auf dem Agarosegel folgende Bedingungen:
GG: 101 + 39 bp
GC: 140 + 101 + 39 bp
CC: 140 bp
Polymorphismen -173 und -179:
Die Genotypisierungen der beiden Polymorphismen nutzen den selben Antisense-Primer: 5'- ACTCGAGCGGGCTAAAGC -3' (SEQ ID NO:5). Die verwendeten Sense-Primer stellen Mutagenese-Primer dar, bei denen eine Base ausgetauscht wurde (unterstrichen), um eine Restriktionsschnittstelle für die Enzyme *BsrDI*(-179, das T-Allel wird geschnitten) bzw. *BslI*(-173, das 6G-Allel wird geschnitten) einzuführen:
   -173: 5'- GCAGCAGCTCCTCTGCGCAA -3' (SEQ ID NO:6)
   -179: 5'- GCAGCAGCTCCTCTGTCCAA -3' (SEQ ID NO:7)

### Beispiel 2: Bestimmung der Verteilung der FNTB-Promotor-Polymorphismen in der Normalbevölkerung und Verwendung der Genotypen zum Auffinden weiterer relevanter Polymorphismen und Haplotypen Hierzu wurden DNA-Proben von gesunden Kaukasiern genotypisiert. Das Ergebnis ist in der folgenden Tabelle 1 dargestellt:

**Tabelle 1: Verteilung der FNTB-Polymorphismen bei Kaukasiern deutschen Ursprungs**

| Genotyp -609 | Kaukasier |
|---|---|
| GG | 70 (40.9%) |
| GC | 73 (42.7%) |
| CC | 28 (16.4%) |
| | |

| Genotyp -179 | Kaukasier |
|---|---|
| TT | 90 (90.0%) |
| TA | 10 (10.0%) |
| AA | 0 |
| | |

| Genotyp -173 | Kaukasier |
|---|---|
| 6G/6G | 99 (57.9%) |
| 6G/5G | 59 (34.5%) |
| 5G/5G | 13 (7.6%) |

### Beispiel 3: Nierenzellkarzinom

Nierenkrebs ist eine schwerwiegende Krankheit. Die Heilungschancen sind abhängig von der Tumorgröße und von der Tumorausbreitung. Bei Patienten ohne Metastasen beträgt die 10-Jahres-Überlebensrate bis zu 80 %, allerdings mit einer erheblichen interindividuellen Variabilität. Durch den heute weit verbreiteten Einsatz von Ultraschalltechnik werden viele der Tumoren bereits in einem frühen nicht metastasierten Stadium erkannt und können so rechtzeitig behandelt werden. Bei einem Vorliegen von Fernmetastasen ist es möglich, die operative Entfernung der Niere mit einer anschließenden Immuntherapie mit Interleukin-2 oder Interferon-Alpha zu kombinieren. Dadurch wird die körpereigene Krebszellabwehr gestärkt. Beim metastasierten Nierenzellkarzinom kann mit der Kombination von Interferon, Interleukin-2 und 5-Fluorouracil in Studien eine Ansprechrate von 36 % erreicht werden. Derzeit gibt es keine prädiktiven Marker für das Überleben bei Patienten mit Nierenzellkarzinom.

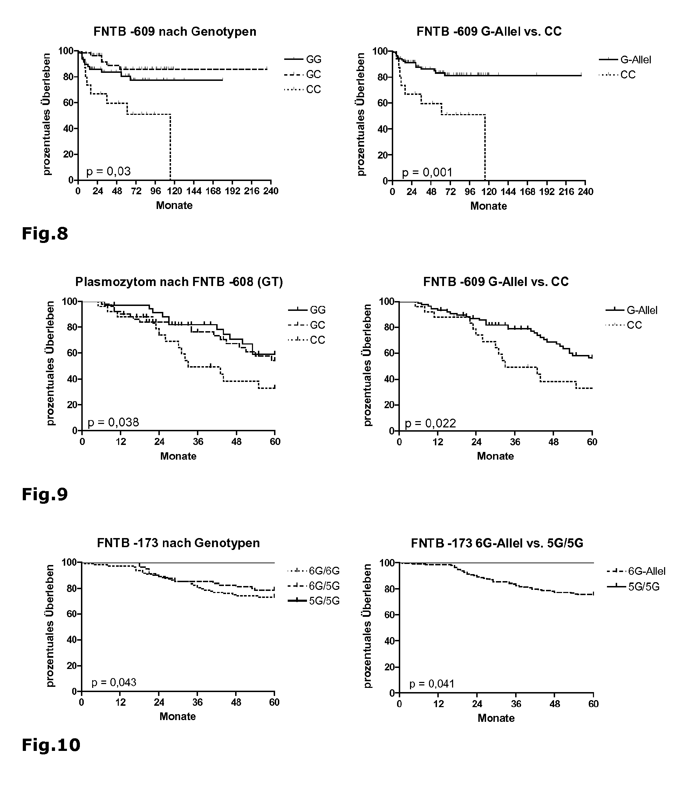

Fig. 8 zeigt das Überleben in Abhängigkeit vom -609 *FNTB*-Genotyp. Patienten mit dem GG- und GC-Genotyp überleben deutlich länger als Patienten mit dem CC-Genotype. Die mediane Zeit bis zum Tod beträgt bei den CC-Trägern 115 Monate. Für Patienten mit dem GG oder GC-Genotyp kann das mediane Überleben nicht angegeben werden, da über den gesamten Beobachtungszeitraum von 240 Monaten weniger als die Hälfte dieser Patienten sterben.

### Beispiel 4: Plasmozytom (Multiples Myelom)

Das Plasmozytom ist eine maligne klonale Neoplasie der terminal differenzierten B-Zellen und tritt am häufigsten zwischen dem 60. und 70. Lebensjahr auf. Männer sind davon etwas häufiger betroffen als Frauen. Das Plasmozytom stellt eine der häufigsten hämatologischen Neoplasien dar, es kann durch äußere Einflüsse hervorgerufen werden. Zu den Risikofaktoren gehören Pestizide und Strahlenexposition des Organismus. Nach Diagnosestellung werden die Patienten in drei Stadien eingeteilt, die auch für die Therapie von Bedeutung sind. Asymptomatische Patienten im Stadium I werden nicht behandelt, aber regelmäßig untersucht. Die Stadien II und III erfordern eine Chemotherapie, wobei entweder eine Hochdosistherapie mit hämatopoetischer Stammzelltransplanation oder eine mildere Chemotherapie z. B. mit Melphalan und Prednison in Frage kommen. Eine Heilung ist durch die Chemotherapie zur Zeit nicht zu erwarten. Aufgabe der Erfindung ist es zu zeigen, dass die Verwendung von Genveränderungen in *FNTB* dazu geeignet ist, den weiteren Verlauf der Erkrankung vorherzusagen.

Fig. 9 zeigt das Überleben von Patienten mit Plasmozytom in Abhängigkeit vom -609 *FNTB*-Genotyp. Hierbei sind deutliche Unterschiede für das Überleben der Patienten zu sehen. Patienten mit dem GG-Genotyp zeigen das beste Überleben, gefolgt vom GC-Genotyp. Die mediane Zeit bis zum Tod beträgt 32 Monate beim CC-Genotyp. Beim GG- und GC-Genotyp kann keine mediane Zeit angegeben werden, da bis zum Ende der Studie weniger als die Hälfte dieser Patienten verstarben.

### Beispiel 5: Mammakarzinom

Das Mammakarzinom ist der häufigste Tumor der weiblichen Bevölkerung in Europa und den USA. Es betrifft 7 -10 % aller Frauen und macht 25 % der gesamten weiblichen Krebsmortalität aus. Die Ätiologie des Mammakarzinoms ist noch nicht bekannt, jedoch sind Risikofaktoren beschrieben, wie eine familiäre Disposition, Strahlenexposition oder Östrogeneinfluss. Bei den meisten Patientinnen ergeben die Untersuchungen, dass es sich um ein invasives Karzinom handelt. Mit wenigen Ausnahmen wird jedes operable Mammakarzinom selbst bei nachgewiesener Fernmetastasierung chirurgisch behandelt. Die unterschiedlich radikale operative Erstversorgung hat Variationen der lokoregionären Rezidivrate, nicht aber der langfristigen Überlebenschance zur Folge. Zudem können Rezidive oder Fernmetastasen nicht selten erst nach 5 oder sogar 10 Jahren manifest werden. Es ist deshalb wichtig, diese Läsionen frühzeitig zu erkennen und für die Patientinnen engmaschig nachzusorgen. Nachsorgeuntersuchungen werden in regelmäßigen Intervallen durchgeführt, bei zwischenzeitlichem Verdacht auch bis zu 10 Jahre postoperativ. Bislang gibt es kaum valide Marker, die für den weiteren Verlauf der Erkrankung prädiktiv sind. Derzeit werden daher die klassischen Faktoren wie Tumorgröße, Metastasierung, Lymphknotenbefall, Hormonrezeptorstatus etc. für die Prognose herangezogen. Genetische Marker für Überlebenswahrscheinlichkeit und Therapieansprechen würden die Betreuung von Patientinnen mit Mammakarzinom wesentlich verbessern. Aufgabe der Erfindung ist es zu zeigen, dass die Verwendung von Genveränderungen in *FNTB* dazu geeignet ist, den weiteren Verlauf der Erkrankung vorherzusagen.

Fig. 10 zeigt das Überleben von Mammakarzinom-Patientinnen in Abhängigkeit vom -173 Genotyp. Patientinnen mit dem 5G/5G-Genotyp zeigen das beste Überleben verglichen mit Patientinnen, die mindestens ein 6G-Allel tragen.

### Beispiel 6: Malignes Melanom

Das maligne Melanom ist ein bösartiger Tumor der Haut, der insgesamt in der weißen Bevölkerung weltweit zunimmt. Die Hauptrisikofaktoren sind ein heller Hauttyp, eine hohe Anzahl von Nävi, rötliche Haare und die Sonnenexposition insbesondere in der Kindheit. Während der solitäre lokale kutane Befund über die sogenannte ABCD-Regel schnell erkannt und chirurgisch therapiert werden kann, weist das metastasierte maligne Melanom eine äußerst ungünstige Prognose auf. Trotz unterschiedlichster Chemotherapie-Ansätze liegt die mediane Überlebenszeit nur bei ca. 6 Monaten. Auch ein Teil der primär kurativ behandelten Melanome rezidiviert, daher wird für diese Patientengruppe eine intensive Nachsorge über 10 Jahre empfohlen. Es stehen bisher nur wenige geeignete Marker zur Verfügung, die für den weiteren Verlauf der Erkrankung prädiktiv sind. Derzeit werden daher die klassischen Faktoren wie Tumorgröße, Eindringtiefe, Metastasierung, Lymphknotenbefall etc. für die Prognose herangezogen. Genetische Marker für Überlebenswahrscheinlichkeit und Therapieansprechen würden die Betreuung von Patienten mit malignem Melanom wesentlich verbessern. Aufgabe der Erfindung ist es zu zeigen, dass die Verwendung von Genveränderungen in *FNTB* dazu geeignet ist, den weiteren Verlauf der Erkrankung vorherzusagen.

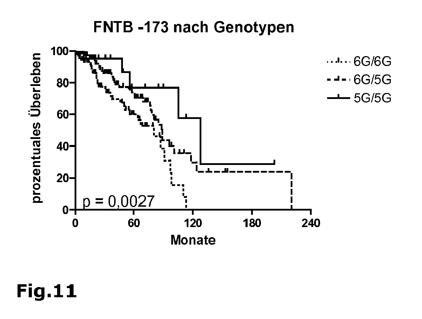

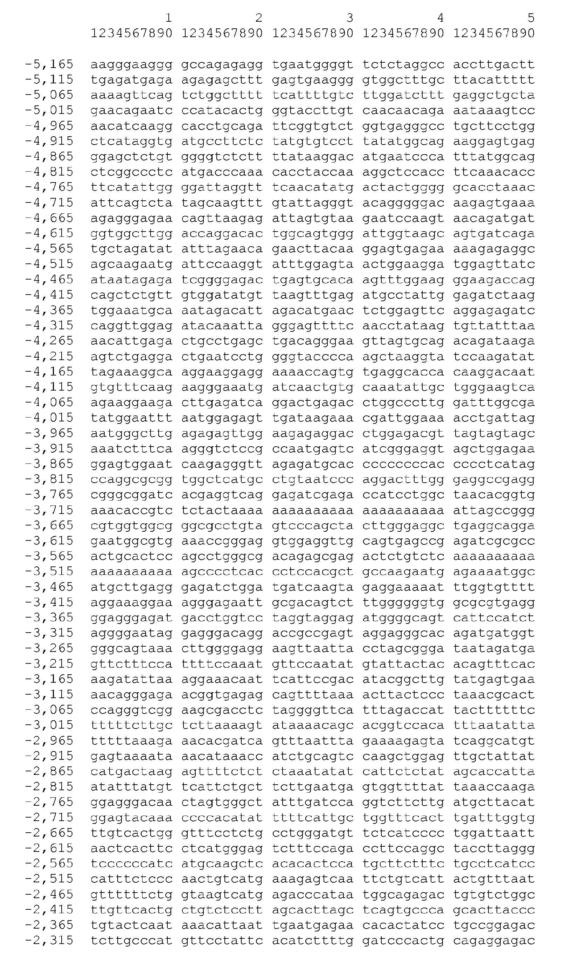

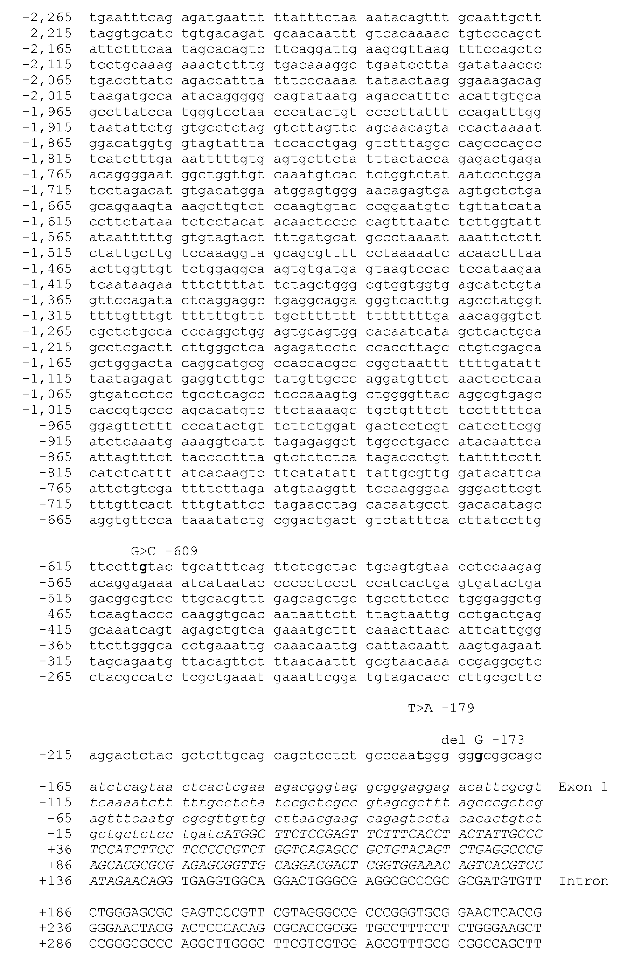

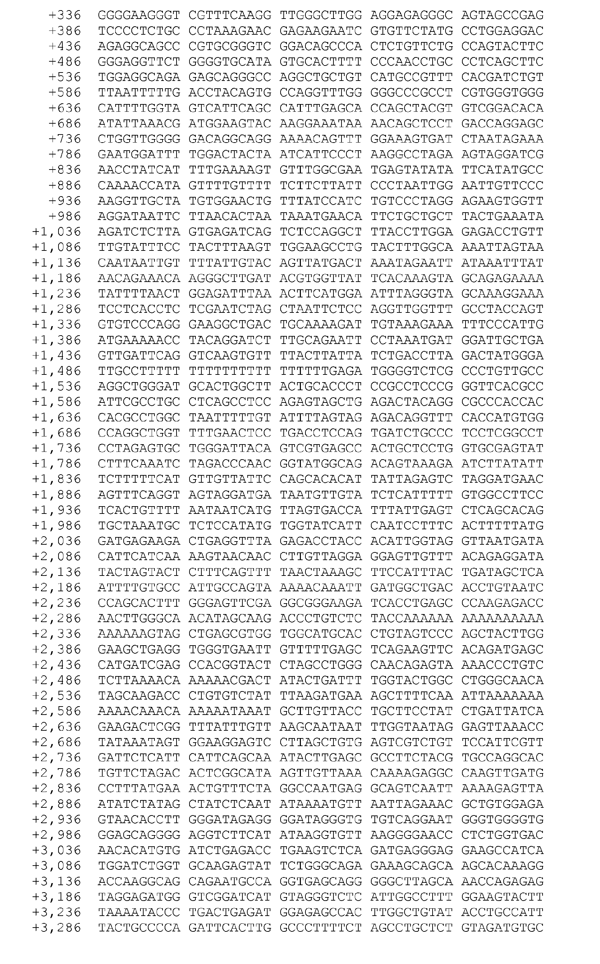

Fig. 11 zeigt das Überleben von Melanom-Patienten in Abhängigkeit vom -173 Genotyp. Patienten mit dem 5G/5G-Genotyp zeigen das beste Überleben mit einem medianen Überleben von 125 Monaten, gefolgt von heterozygoten Patienten mit einem medianen Überleben von 90 Monaten. Patienten mit dem 6G/6G-Genotyp zeigen das schlechteste mediane Überleben mit 80 Monaten.

**Seqenzprotokoll, freier Text**

| | |
|---|---|
| SEQ ID NO:1 | putative Promoterregion für das Gen *FNTB* |
| SEQ ID NO:2 | Promotersequenz von *FNTB* mit flankierenden Sequenzen |
| SEQ ID NOs:3 bis 9 | Primer |

## Patentansprüche

1. *In*-*vitro* Verfahren zur Vorhersage von Krankheitsrisiken, Krankheitsverläufen, Arzneimittelrisiken und zum Auffinden von Drug Targets, das das Durchmustern einer biologischen Probe nach einer oder mehreren Genveränderung(en) in der Promotorregion des Gens *FNTB,* das für die β-Untereinheit des Proteins Farnesyltransferase kodiert, auf dem Humanchromosom 14q23-24 umfasst.

2. Verfahren nach Anspruch 1, wobei die biologische Probe nach einem oder mehreren der Polymorphismen -609, -179 oder -173 der in Fig. 12 gezeigten Sequenz in der Promotorregion des Gens *FNTB* durchmustert wird, wobei bei diesen Polymorphismen bei Position -609 ein Guanin durch ein Cytosin, bei Position -179 ein Thymin durch ein Adenin substituiert ist und bei Position - 173 ein Guanin deletiert ist.

3. Verfahren nach Anspruch 1 oder 2, wobei das Durchmustern durch direkte Sequenzierung, PCR mit nachfolgender Restriktionsanalyse, reverse Hybridisierung, Dot-blot- oder slot-blot-Verfahren, Massenspektrometrie, Taqman®-oder Light-Cycler®-Technologie, Pyrosequencing®, Invader®-Technologie, Luminex-Verfahren und Umsetzung mit DNA-Chips erfolgt.

4. Verfahren nach Anspruch 3, das eine Amplifikationsreaktion von mit einem Primerpaar und gegebenenfalls zusätzlichen Sequenzierprimern, die wenigstens 5 zusammenhängende Nukleotide aus den Nukleotiden -5000 bis +5000, vorzugsweise aus den Nukleotiden -2609 bis +1391 für den SNP -609, -2179 bis +1821 für den SNP -179 und -2173 bis +1827 für den SNP -173 der in Fig. 12 gezeigten Sequenz, wobei die Primerpaare den jeweiligen Polymorphismus einschließen und der Nachweis auch gemeinsam erfolgen kann, umfasst.

5. Verfahren nach Anspruch 3 oder 4, das aus einen oder mehrere der folgenden Verfahren ausgewählt ist
(i) direkte Sequenzierung in genomischer DNA, umfassend Umsetzung mit wenigstens einem Primerpaar und gegebenenfalls zusätzlichen Sequenzierprimern, die wenigstens 10 zusammenhängende Nukleotide, aus den Nukleotiden -5000 und +5000, vorzugsweise aus den Nukleotiden -2609 bis +1391 für den SNP -609, -2179 bis +1821 für den SNP -179 und -2173 bis +1827 für den SNP -173 der in Fig. 12 gezeigten Sequenz, wobei die Primerpaare den jeweiligen Polymorphismus einschließen und der Nachweis auch gemeinsam erfolgen kann, umfassen;
(ii) Nachweis in genomischer DNA mittels MALDI-TOF-MS, umfassend PCR mit wenigstens einem Primerpaar, das wenigstens wenigstens 10 zusammenhängende Nukleotide, aus den Nukleotiden -5000 und +5000, vorzugsweise aus den Nukleotiden -2609 bis +1391 für den SNP -609, -2179 bis +1821 für den SNP -179 und -2173 bis +1827 für den SNP -173 der in Fig. 12 gezeigten Sequenz, wobei die Primerpaare den jeweiligen Polymorphismus einschließen und der Nachweis auch gemeinsam erfolgen kann, umfasst, Extension mit Primern, die wenigstens 10 zusammenhängende Nukleotide umfassen, die einer Sequenz in 5'-Richtung oder in 3'-Richtung ausgehend von den jeweiligen Polymorphismus-Positionen in der in Fig. 12 gezeigten Sequenz entsprechen, und Genotypisierung des Polymorphismus über die unterschiedlichen Nukleotidmassen;
(iii) Nachweis in genomischer DNA mittels Restriktionsfragment-Längenpolymorphismus (RFLP), umfassend PCR mit wenigstens einem Primerpaar, das wenigstens 10 zusammenhängende Nukleotide aus den Nukleotiden -5000 und +5000, vorzugsweise aus den Nukleotiden -2609 bis +1391 für den SNP -609, -2179 bis +1821 für den SNP -179 und -2173 bis +1827 für den SNP - 173 der in Fig. 12 gezeigten Sequenz, wobei die Primerpaare den jeweiligen Polymorphismus einschließen und der Nachweis auch gemeinsam erfolgen kann, umfasst, optional in Gegenwart eines Mutageneseprimers, und RFLP Bestimmung;
(iv) Nachweis des Polymorphismus in genomischer DNA mittels Allelspezifischer PCR, umfassend Umsetzung mit wenigstens einem Primerpaar, das wenigstens 10 zusammenhängenden Nukleotiden, aus den Nukleotiden -5000 und +5000, vorzugsweise aus den Nukleotiden -2609 bis +1391 für den SNP -609, -2179 bis +1821 für den SNP -179 und -2173 bis +1827 für den SNP - 173 der in Fig. 12 gezeigten Sequenz, wobei die Primerpaare den jeweiligen Polymorphismus einschließen und der Nachweis auch gemeinsam erfolgen kann, umfasst, wobei jeweils ein Primer spezifisch für die jeweiligen Allele an den Position -609, -179 und -173 ist;
(v) Nachweis des Polymorphismus in genomischer DNA mittels HPLC, umfassend PCR mit wenigstens einem Primerpaar, das wenigstens 10 zusammenhängende Nukleotide aus den Nukleotiden -5000 und +5000, vorzugsweise aus den Nukleotiden -2609 bis +1391 für den SNP -609, -2179 bis +1821 für den SNP -179 und -2173 bis +1827 für den SNP -173 der in Fig. 12 gezeigten Sequenz, wobei die Primerpaare den jeweiligen Polymorphismus einschließen und der Nachweis auch gemeinsam erfolgen kann, umfasst, Aufschmelzen der Amplifikate und HPLC Nachweis über die Bildung von Heteroduplexen;
(vi) Nachweis des Polymorphismus in genomischer DNA mittels allelspezifischer Hybridisierung, umfassend Umsetzung mit allelspezifischen Sonden und optional vorrausgehende PCR Amplifikation der genomischen DNA mit wenigstens einem Primerpaar, das wenigstens 10 zusammenhängenden Nukleotiden, aus den Nukleotiden -5000 und +5000, vorzugsweise aus den Nukleotiden - 2609 bis +1391 für den SNP -609, -2179 bis +1821 für den SNP -179 und - 2173 bis +1827 für den SNP -173 der in Fig. 12 gezeigten Sequenz, wobei die Primerpaare den jeweiligen Polymorphismus einschließen und der Nachweis auch gemeinsam erfolgen kann" wobei die allelspezifischen Sonden eine Sequenz von wenigstens 10 zusammenhängenden Nukleotiden, aus den Nukleotiden -5000 und +5000, vorzugsweise aus den Nukleotiden -2609 bis +1391 für den SNP -609, -2179 bis +1821 für den SNP -179 und -2173 bis +1827 für den SNP -173 der in Fig. 12 gezeigten Sequenz, wobei die Primerpaare den jeweiligen Polymorphismus einschließen und der Nachweis auch gemeinsam erfolgen kann, und wobei die Sonden spezifisch für das jeweilige Allel der Polymorphismen an genomischer Position -609, -179 und/oder -173 sind.

6. Verfahren nach Anspruch 5, wobei
(i) die Primer wenigstens 15, vorzugsweise 15 bis 30 zusammenhängende Nukleotide der Sequenzen von Fig. 12 aufweisen und vorzugsweise die Sequenz von SEQ ID NOs:3 bis 9, Nukleotide -2609 bis +1391 für den SNP - 609, -2179 bis +1821 für den SNP -179 und -2173 bis +1827 für den SNP - 173 der in Fig. 12 gezeigten Sequenz aufweisen; und/oder
(ii) die Sonden wenigstens 20 zusammenhängende Nukleotide der Sequenzen von Fig. 12 aufweisen.

7. Verfahren nach einem oder mehreren der Ansprüche 1 bis 6,
(i) das zur Voraussage von Risiken oder dem Verlauf einer Erkrankung, einschließlich Nierenzellkarzinom, Plasmozytom, Mammakarzinom und malignes Melanom geeignet ist, wobei bei -609 der Genotyp GG, bei -179 der Genotyp TT und bei -173 der Genotyp 5G/5G die beste Überlebensprognose zeigt; oder
(ii) das zum Auffinden und Evaluieren von Krebstherapeutika geeignet ist; oder
(iii) in dem Hemmstoffe der Farnesyltransferase, insbesondere Tipifarnib, Lonafarnib, BMS-214662, L778123 oder ABT-100 eingesetzt werden.

8. Verfahren zum Auffinden relevanter Polymorphismen und Haplotypen, im Gen *FNTB* und in dazu benachbarten Genen, umfassend die Quantifizierung der Expression von FNTB im Zusammenhang mit dem in Anspruch 1 oder 2 beschriebenen Polymorphismus und Abgleich mit der Expression von anderen Polymorphismen der *FNTB.*

9. Kit zur Durchführung des Verfahrens nach Anspruch 1 bis 7, umfassend Primer und/oder Sonden, die zur Detektion der Polymorphismen -609, -179 und/oder -173 der in Fig. 12 gezeigten Sequenz in der Promotorregion des Gens *FNTB* geeignet sind.

10. Kit nach Anspruch 9, wobei
(i) die Primer und Sonden wie in Anspruch 4 bis 6 beschrieben sind, und/oder
(ii) der Kit weiterhin Restriktionsenzyme, Polymerasen, Label, Vergleichssubstanzen, Puffer usw. enthält.


SEQUENCE LISTING

<110>  Universitaet Duisburg-Essen

<120>  FNTB Polymorphismus

<130>  080016ep

<160>  9

<170>  PatentIn version 3.3

<210>  1
<211>  10250
<212>  DNA
<213>  Homo sapiens

<400>  1
   aagggaaggg gccagagagg tgaatggggt tctctaggcc accttgactt tgagatgaga      60
   agagagcttt gagtgaaggg gtggctttgc ttacattttt aaaagttcag tctggctttt     120
   tcattttgtc ttggatcttt gaggctgcta gaacagaatc ccatacactg ggtaccttgt     180
   caacaacaga aataaagtcc aacatcaagg cacctgcaga ttcggtgtct ggtgagggcc     240
   tgcttcctgg ctcataggtg atgccttctc tatgtgtcct tatatggcag aaggagtgag     300
   ggagctctgt ggggtctctt ttataaggac atgaatccca tttatggcag ctcggccctc     360
   atgacccaaa cacctaccaa aggctccacc ttcaaacacc ttcatattgg ggattaggtt     420
   tcaacatatg actactgggg gcacctaaac attcagtcta tagcaagttt gtattagggt     480
   acagggggac aagagtgaaa agagggagaa cagttaagag attagtgtaa gaatccaagt     540
   aacagatgat ggtggcttgg accaggacac tggcagtggg attggtaagc agtgatcaga     600
   tgctagatat atttagaaca gaacttacaa ggagtgagaa aaagagaggc agcaagaatg     660
   attccaaggt atttggagta actggaagga tggagttatc ataatagaga tcggggagac     720
   tgagtgcaca agtttggaag ggaagaccag cagctctgtt gtggatatgt taagtttgag     780
   atgcctattg gagatctaag tggaaatgca aatagacatt agacatgaac tctggagttc     840
   aggagagatc caggttggag atacaaatta gggagttttc aacctataag tgttatttaa     900
   aacattgaga ctgcctgagc tgacagggaa gttagtgcag acagataaga agtctgagga     960
   ctgaatcctg gggtacccca agctaaggta tccaagatat tagaaaggca aggaaggagg    1020
   aaaaccagtg tgaggcacca caaggacaat gtgtttcaag aagggaaatg atcaactgtg    1080
   caaatattgc tgggaagtca agaaggaaga cttgagatca ggactgagac ctggcccttg    1140
   gatttggcga tatggaattt aatggagagt tgataagaaa cgattggaaa acctgattag    1200
   aatgggcttg agagagttgg aagagaggac ctggagacgt tagtagtagc aaatctttca    1260
   agggtctccg ccaatgagtc atcgggaggt agctggagaa ggagtggaat caagagggtt    1320
   agagatgcac ccccccccac cccctcatag ccaggcgcgg tggctcatgc ctgtaatccc    1380
   aggactttgg gaggccgagg cgggcggatc acgaggtcag gagatcgaga ccatcctggc 1440
   taacacggtg aaacaccgtc tctactaaaa aaaaaaaaaa aaaaaaaaaa attagccggg 1500
   cgtggtggcg ggcgcctgta gtcccagcta cttgggaggc tgaggcagga gaatggcgtg 1560
   aaaccgggag gtggaggttg cagtgagccg agatcgcgcc actgcactcc agcctgggcg 1620
   acagagcgag actctgtctc aaaaaaaaaa aaaaaaaaaa agcccctcac cctccacgct 1680
   gccaagaatg agaaaatggc atgcttgagg gagatctgga tgatcaagta gaggaaaaat 1740
   ttggtgtttt aggaaaggaa agggagaatt gcgacagtct ttggggggtg gcgcgtgagg 1800
   ggagggagat gacctggtcc taggtaggag atggggcagt cattccatct aggggaatag 1860
   gagggacagg accgccgagt aggagggcac agatgatggt gggcagtaaa cttggggagg 1920
   aagttaatta cctagcggga taatagatga gttctttcca ttttccaaat gttccaatat 1980
   gtattactac acagtttcac aagatattaa aggaaacaat tcattccgac atacggcttg 2040
   tatgagtgaa aacagggaga acggtgagag cagttttaaa acttactccc taaacgcact 2100
   ccagggtcgg aagcgacctc taggggttca tttagaccat tacttttttc tttttcttgc 2160
   tcttaaaagt ataaaacagc acggtccaca tttaatatta tttttaaaga aacacgatca 2220
   gtttaattta gaaaagagta tcaggcatgt gagtaaaata aacataaacc atctgcagtc 2280
   caagctggag ttgctattat catgactaag agttttctct ctaaatatat cattctctat 2340
   agcaccatta atatttatgt tcattctgct tcttgaatga gtggttttat taaaccaaga 2400
   ggagggacaa ctagtgggct atttgatcca ggtcttcttg atgcttacat ggagtacaaa 2460
   ccccacatat ttttcattgc tggtttcact tgatttggtg ttgtcactgg gtttcctctg 2520
   cctgggatgt tctcatcccc tggattaatt aactcacttc ctcatgggag tctttccaga 2580
   ccttccaggc taccttaggg tccccccatc atgcaagctc acacactcca tgcttctttc 2640
   tgcctcatcc catttctccc aactgtcatg aaagagtcaa ttctgtcatt actgtttaat 2700
   gttttttctg gtaagtcatg agacccataa tggcagagac tgtgtctggc ttgttcactg 2760
   ctgtctcctt agcacttagc tcagtgccca gcacttaccc tgtactcaat aaacattaat 2820
   tgaatgagaa cacactatcc tgccggagac tcttgcccat gttcctattc acatcttttg 2880
   gatcccactg cagaggagac tgaatttcag agatgaattt ttatttctaa aatacagttt 2940
   gcaattgctt taggtgcatc tgtgacagat gcaacaattt gtcacaaaac tgtcccagct 3000
   attctttcaa tagcacagtc ttcaggattg aagcgttaag tttccagctc tcctgcaaag 3060
   aaactctttg tgacaaaggc tgaatcctta gatataaccc tgaccttatc agaccattta 3120
   tttcccaaaa tataactaag ggaaagacag taagatgcca atacaggggg cagtataatg 3180
   agaccatttc acattgtgca gccttatcca tgggtcctaa cccatactgt ccccttattt 3240
   ccagatttgg taatattctg gtgcctctag gtcttagttc agcaacagta ccactaaaat 3300
   ggacatggtg gtagtattta tccacctgag gtctttaggc cagcccagcc tcatctttga 3360
   aatttttgtg agtgcttcta tttactacca gagactgaga acaggggaat ggctggttgt 3420
   caaatgtcac tctggtctat aatccctgga tcctagacat gtgacatgga atggagtggg 3480
   aacagagtga agtgctctga gcaggaagta aagcttgtct ccaagtgtac ccggaatgtc 3540
   tgttatcata ccttctataa tctcctacat acaactcccc cagtttaatc tcttggtatt 3600
   ataatttttg gtgtagtact tttgatgcat gccctaaaat aaattctctt ctattgcttg 3660
   tccaaaggta gcagcgtttt cctaaaaatc acaactttaa acttggttgt tctggaggca 3720
   agtgtgatga gtaagtccac tccataagaa tcaataagaa tttcttttat tctagctggg 3780
   cgtggtggtg agcatctgta gttccagata ctcaggaggc tgaggcagga gggtcacttg 3840
   agcctatggt ttttgtttgt ttttttgttt tgcttttttt tttttttgga aacaggtctt 3900
   cgctctgcca cccaggctgg agtgcagtgg cacaatcata gctcactgca gcctcgactt 3960
   cttgggctca agagatcctc ccaccttagc ctgtcgagca gctgggacta caggcatgcg 4020
   ccaccacgcc cggctaattt ttttgatatt taatagagat gaggtcttgc tatgttgccc 4080
   aggatgttct aactcctcaa gtgatcctcc tgcctcagcc tcccaaagtg ctggggttac 4140
   aggcgtgagc caccgtgccc agcacatgtc ttctaaaagc tgctgtttct tcctttttca 4200
   ggagttcttt cccatactgt tcttctggat gactcctcgt catccttcgg atctcaaatg 4260
   aaaggtcatt tagagaggct tggcctgacc atacaattca attagtttct taccccttta 4320
   gtctctctca tagaccctgt tattttcctt catctcattt atcacaagtc ttcatatatt 4380
   tattgcgttg gatacattca attctgtcga ttttcttaga atgtaaggtt tccaagggaa 4440
   gggacttcgt tttgttcact tttgtattcc tagaacctag cacaatgcct gacacatagc 4500
   aggtgttcca taaatatctg cggactgact gtctatttca cttatccttg ttccttgtac 4560
   tgcatttcag ttctcgctac tgcagtgtaa cctccaagag acaggagaaa atcataatac 4620
   ccccctccct ccatcactga gtgatactga gacggcgtcc ttgcacgttt gagcagctgc 4680
   tgccttctcc tgggaggctg tcaagtaccc caaggtgcac aataattctt ttagtaattg 4740
   cctgactgag gcaaatcagt agagctgtca gaaatgcttt caaacttaac attcattggg 4800
   ttcttgggca cctgaaattg caaacaattg cattacaatt aagtgagaat tagcagaatg 4860
   ttacagttct ttaacaattt gcgtaacaaa ccgaggcgtc ctacgccatc tcgctgaaat 4920
   gaaattcgga tgtagacacc cttgcgcttc aggactctac gctcttgcag cagctcctct 4980
   gcccaatggg gggcggcagc atctcagtaa ctcactcgaa agacgggtag gcgggaggag 5040
   acattcgcgt tcaaaatctt tttgcctcta tccgctcgcc gtagcgcttt agcccgctcg 5100
   agtttcaatg cgcgttgttg cttaacgaag cagagtccta cacactgtct gctgctctcc 5160
   tgatcatggc ttctccgagt tctttcacct actattgccc tccatcttcc tcccccgtct 5220
   ggtcagagcc gctgtacagt ctgaggcccg agcacgcgcg agagcggttg caggacgact 5280
   cggtggaaac agtcacgtcc atagaacagg tgaggtggca ggactgggcg aggcgcccgc 5340
   gcgatgtgtt ctgggagcgc gagtcccgtt cgtagggccg cccgggtgcg gaactcaccg 5400
   gggaactacg actcccacag cgcaccgcgg tgcctttcct ctgggaagct ccgggcgccc 5460
   aggcttgggc ttcgtcgtgg agcgtttgcg cggccagctt ggggaagggt cgtttcaagg 5520
   ttgggcttgg aggagagggc agtagccgag tcccctctgc cctaaagaac gagaagaatc 5580
   gtgttctatg cctggaggac agaggcagcc cgtgcgggtc ggacagccca ctctgttctg 5640
   ccagtacttc gggaggttct ggggtgcata gtgcactttt cccaacctgc cctcagcttc 5700
   tggaggcaga gagcagggcc aggctgctgt catgccgttt cacgatctgt ttaatttttg 5760
   acctacagtg ccaggtttgg gggcccgcct cgtgggtggg cattttggta gtcattcagc 5820
   catttgagca ccagctacgt gtcggacaca atattaaacg atggaagtac aaggaaataa 5880
   aacagctcct gaccaggagc ctggttgggg gacaggcagg aaaacagttt ggaaagtgat 5940
   ctaatagaaa gaatggattt tggactacta atcattccct aaggcctaga agtaggatcg 6000
   aacctatcat tttgaaaagt gtttggcgaa tgagtatata ttcatatgcc caaaaccata 6060
   gttttgtttt tcttcttatt ccctaattgg aattgttccc aaggttgcta tgtggaactg 6120
   tttatccatc tgtccctagg agaagtggtt aggataattc ttaacactaa taaatgaaca 6180
   ttctgctgct tactgaaata agatctctta gtgagatcag tctccaggct ttaccttgga 6240
   gagacctgtt ttgtatttcc tactttaagt tggaagcctg tactttggca aaattagtaa 6300
   caataattgt tttattgtac agttatgact aaatagaatt ataaatttat aacagaaaca 6360
   agggcttgat acgtggttat tcacaaagta gcagagaaaa tattttaact ggagatttaa 6420
   acttcatgga atttagggta gcaaaggaaa tcctcacctc tcgaatctag ctaattctcc 6480
   aggttggttt gcctaccagt gtgtcccagg gaaggctgac tgcaaaagat tgtaaagaaa 6540
   tttcccattg atgaaaaacc tacaggatct ttgcagaatt cctaaatgat ggattgctga 6600
   gttgattcag gtcaagtgtt ttacttatta tctgacctta gactatggga ttgccttttt 6660
   tttttttttt ttttttgaga tggggtctcg ccctgttgcc aggctgggat gcactggctt 6720
   actgcaccct ccgcctcccg ggttcacgcc attcgcctgc ctcagcctcc agagtagctg 6780
   agactacagg cgcccaccac cacgcctggc taatttttgt attttagtag agacaggttt 6840
   caccatgtgg ccaggctggt tttgaactcc tgacctccag tgatctgccc tcctcggcct 6900
   cctagagtgc tgggattaca gtcgtgagcc actgctcctg gtgcgagtat ctttcaaatc 6960
   tagacccaac ggtatggcag acagtaaaga atcttatatt tctttttcat gttgttattc 7020
   cagcacacat tattagagtc taggatgaac agtttcaggt agtaggatga taatgttgta 7080
   tctcattttt gtggccttcc tcactgtttt aataatcatg ttagtgacca tttattgagt 7140
   ctcagcacag tgctaaatgc tctccatatg tggtatcatt caatcctttc actttttatg 7200
   gatgagaaga ctgaggttta gagacctacc acattggtag gttaatgata cattcatcaa 7260
   aagtaacaac cttgttagga ggagttgttt acagaggata tactagtact ctttcagttt 7320
   taactaaagc ttccatttac tgatagctca attttgtgcc attgccagta aaaacaaatt 7380
   gatggctgac acctgtaatc ccagcacttt gggagttcga ggcgggaaga tcacctgagc 7440
   ccaagagacc aacttgggca acatagcaag accctgtctc taccaaaaaa aaaaaaaaaa 7500
   aaaaaagtag ctgagcgtgg tggcatgcac ctgtagtccc agctacttgg gaagctgagg 7560
   tgggtgaatt gtttttgagc tcagaagttc acagatgagc catgatcgag ccacggtact 7620
   ctagcctggg caacagagta aaaccctgtc tcttaaaaca aaaaacgact atactgattt 7680
   tggtactggc ctgggcaaca tagcaagacc ctgtgtctat ttaagatgaa agcttttcaa 7740
   attaaaaaaa aaaacaaaca aaaaataaat gcttgttacc tgcttcctat ctgattatca 7800
   gaagactcgg tttatttgtt aagcaataat ttggtaatag gagttaaacc tataaatagt 7860
   ggaaggagtc cttagctgtg agtcgtctgt tccattcgtt gattctcatt cattcagcaa 7920
   atacttgagc gccttctacg tgccaggcac tgttctagac actcggcata agttgttaaa 7980
   caaaagaggc caagttgatg cctttatgaa actgtttcta ggccaatgag gcagtcaatt 8040
   aaaagagtta atatctatag ctatctcaat ataaaatgtt aattagaaac gctgtggaga 8100
   gtaacacctt gggatagagg ggatagggtg tgtcaggaat gggtggggtg ggagcagggg 8160
   aggtcttcat ataaggtgtt aaggggaacc ctctggtgac aacacatgtg atctgagacc 8220
   tgaagtctca gatgagggag gaagccatca tggatctggt gcaagagtat tctgggcaga 8280
   gaaagcagca agcacaaagg accaaggcag cagaatgcca ggtgagcagg gggcttagca 8340
   aaccagagag taggagatgg gtcggatcat gtagggtctc attggccttt ggaagtactt 8400
   taaaataccc tgactgagat ggagagccac ttggctgtat acctgccatt tactgcccca 8460
   gattcacttg gcccttttct agcctgctct gtagatgtgc cccaggaggg ggccctgcac 8520
   tgacaggttg ccttgcttta tggctgcggg ccatgggaag tattgctggg ggtgaccgag 8580
   aaggagaaga atgagaccac tgtatttctt ccctcctgca ggcttgtgtg gggtggggtt 8640
   tccttagact taaggtcaca tttcctgtca tgtgccttct ctgcggagct ctttgtctcc 8700
   ctccctctcc atccttcaca ccttcaggtc tatgaggagt tcaggtaccc ctgatttacc 8760
   agctccagat actgcatttt ctcttgtggt ttccctacac ctgcccacat ctttgtaaat 8820
   agttccttta taaactctcc tcaaatcacc cactttgtgc catctgtttc ctgccaggac 8880
   ttagactgat ccatgatcag agagttttga gcaggggagt gacacagtct gactagcatt 8940
   ttaacaggct catttaggtt actgtcaaga gtagatggtg gggagtgagg gtggatacag 9000
   ggaaaccagt taggaggcta ttgcagtatc ctgggagaga ggtgacggtg gcttagctca 9060
   cagtgacggt agtggaggta atgagaaatg gtcagctctg gatattttta tgaaggtctc 9120
   taccttgcta agctgttttg ttaatgcaga tgcagggagg gcctttcatg acactagaga 9180
   acaagacatg gttgtattgt tagtgcctgc cttccttatt gtgccatctg gaatcatccc 9240
   attgcagaca ctctgattca ggccagtagg ttaatactga tagtctcaga ggtagcagcc 9300
   ttgccaaaaa caggtgttta caaaagatac tactgttttt ctttgagttt gtctttgaac 9360
   agaggcaaac gcgtcctcca ctttaaatgg catggtgtca ttgaaccatt ggcatggttc 9420
   attgtcaatg aagtgacttg aactctgagg ggaatataag agaattatta gtcttgatcc 9480
   ctgccctcaa aattctttta tcttagagag ataaaaaaga atatgctgta aggccgagtg 9540
   cggtggctca ctcctgtaat cccagcactt tgggaggctg aggctggcgg atcactggag 9600
   gtcaggagtt tgagaccagc ctagccaaca tggtgaaacc tcatctctac taaaaaaata 9660
   caaaaattag ctaggcgtgg tggtgtgcac ctgtaatacc agctactcag gacactgagg 9720
   caggtgaatc tctttaacct gggaggcgga ggttgcagtg agccgagatt gcaccattgc 9780
   actcaagcct gggtgacaga gcaagactcc atctcaaaaa agaaaaaaat aagaagaata 9840
   tgctataggc agtcacagag tgctaaatta taactgcttt gcacgaggat tttggggagt 9900
   tgaaaagcca tctgtgtggg ctgaggtggt caggatgact tcatggagaa cgtggaactg 9960
   ggtgggctgt aaaaaataga gtttggggca gccattcaag gagggagagc tgttttgagg 10020
   gaagactcag gcttgactgg gggcgttaag atggcttacc aggaacggag ggtgcaggtt 10080
   ggatgtggta ggaaatgagg tgagctgggc gggtgggccc ctatcagggt attaagaggc 10140
   aggtgatgtg ctggcttcag gttcgggcag tgggccacca catatcctaa ggagggacca 10200
   gcggaacaga atcctgacct gctgttgact tatttcagct tataggatcc 10250

<210> 2
<211> 1050
<212> DNA
<213> Homo sapiens

<400> 2
atctcaaatg aaaggtcatt tagagaggct tggcctgacc atacaattca attagtttct 60

taccccttta gtctctctca tagaccctgt tattttcctt catctcattt atcacaagtc 120

ttcatatatt tattgcgttg gatacattca attctgtcga ttttcttaga atgtaaggtt 180

tccaagggaa gggacttcgt tttgttcact tttgtattcc tagaacctag cacaatgcct 240

gacacatagc aggtgttcca taaatatctg cggactgact gtctatttca cttatccttg 300

ttccttgtac tgcatttcag ttctcgctac tgcagtgtaa cctccaagag acaggagaaa 360

atcataatac ccccctccct ccatcactga gtgatactga gacggcgtcc ttgcacgttt    420

gagcagctgc tgccttctcc tgggaggctg tcaagtaccc caaggtgcac aataattctt    480

ttagtaattg cctgactgag gcaaatcagt agagctgtca gaaatgcttt caaacttaac    540

attcattggg ttcttgggca cctgaaattg caaacaattg cattacaatt aagtgagaat    600

tagcagaatg ttacagttct ttaacaattt gcgtaacaaa ccgaggcgtc ctacgccatc    660

tcgctgaaat gaaattcgga tgtagacacc cttgcgcttc aggactctac gctcttgcag    720

cagctcctct gcccaatggg gggcggcagc atctcagtaa ctcactcgaa agacgggtag    780

gcgggaggag acattcgcgt tcaaaatctt tttgcctcta tccgctcgcc gtagcgcttt    840

agcccgctcg agtttcaatg cgcgttgttg cttaacgaag cagagtccta cacactgtct    900

gctgctctcc tgatcatggc ttctccgagt tctttcacct actattgccc tccatcttcc    960

tcccccgtct ggtcagagcc gctgtacagt ctgaggcccg agcacgcgcg agagcggttg   1020

caggacgact cggtggaaac agtcacgtcc                                    1050

<210>  3
<211>  19
<212>  DNA
<213>  Artificial

<220>
<223>  primer

<400>  3
gcggactgac tgtctattt                                                  19

<210>  4
<211>  20
<212>  DNA
<213>  Artificial

<220>
<223>  primer

<400>  4
gacgccgtct ctcagtatca                                                 20

<210>  5
<211>  18
<212>  DNA
<213>  Artificial

<220>
<223>  primer

<400>  5
actcgagcgg gctaaagc                                                   18

<210>  6

<211>  20
<212>  DNA
<213>  Artificial

<220>
<223>  primer

<400>  6
gcagcagctc ctctgcgcaa                                                    20


<210>  7
<211>  20
<212>  DNA
<213>  Artificial

<220>
<223>  primer

<400>  7
gcagcagctc ctctgtccaa                                                    20


<210>  8
<211>  27
<212>  DNA
<213>  Artificial

<220>
<223>  primer

<400>  8
atactgttct tctggatgac tcctcgt                                            27


<210>  9
<211>  21
<212>  DNA
<213>  Artificial

<220>
<223>  primer

<400>  9
tgtttccacc gagtcgtcct g                                                  21